# EUROPEAN PATENT APPLICATION

(11) **EP 4 414 033 A2**
(43) Date of publication of application: **14.08.2024**
(21) Application number: 24170617.5
(22) Date of filing: 06.02.2020
(51) Int. Cl.: A61P 35/00

(54) **TREATMENT INVOLVING CAR-ENGINEERED T CELLS AND CYTOKINES**

(30) Priority: 08.02.2019 WO PCT/EP2019/053144
(62) Divisional of application: 20702490.2
(71) Applicant: BIONTECH CELL & GENE THERAPIES GMBH, 55131 Mainz (DE)
(72) Inventor: SAHIN, Ugur, 55131 Mainz (DE); OEHM, Petra, 55131 Mainz (DE); RENGSTL, Benjamin, 55131 Mainz (DE); REINHARD, Katharina, 55131 Mainz (DE)
(74) Representative: Graf, Roland

(57) **Abstract**

The present disclosure relates to methods and agents for enhancing the effect of T cells engineered to express chimeric antigen receptors (CARs). These methods and agents are, in particular, useful for the treatment of diseases characterized by diseased cells expressing an antigen the CAR is directed to.

Specifically, the present disclosure relates to methods comprising providing to a subject T cells genetically modified to express a chimeric antigen receptor (CAR) and administering to the subject IL2 or a polynucleotide encoding IL2. The methods of the disclosure may comprise administering IL2 or a polynucleotide encoding IL2 and a further cytokine or a polynucleotide encoding a further cytokine, wherein the further cytokine may be IL7 or IL21. The T cells genetically modified to express a CAR may be provided to the subject by administering the T cells genetically modified to express a CAR or by generating the T cells genetically modified to express a CAR in the subject. The methods of the disclosure may further comprise administering to the subject an antigen or a variant thereof, or a polynucleotide encoding an antigen or a variant thereof, wherein the T cell genetically modified to express a CAR are targeted to the antigen. In one particularly preferred embodiment, the polynucleotides administered according to the present disclosure are RNA.

## Description

### Technical Field

The present disclosure relates to methods and agents for enhancing the effect of T cells engineered to express chimeric antigen receptors (CARs). These methods and agents are, in particular, useful for the treatment of diseases characterized by diseased cells expressing an antigen the CAR is directed to. Specifically, the present disclosure relates to methods comprising providing to a subject T cells genetically modified to express a chimeric antigen receptor (CAR) and administering to the subject IL2 or a polynucleotide encoding IL2. The methods of the disclosure may comprise administering IL2 or a polynucleotide encoding IL2 and a further cytokine or a polynucleotide encoding a further cytokine, wherein the further cytokine may be IL7 or IL21. The T cells genetically modified to express a CAR may be provided to the subject by administering the T cells genetically modified to express a CAR or by generating the T cells genetically modified to express a CAR in the subject. The methods of the disclosure may further comprise administering to the subject an antigen or a variant thereof, or a polynucleotide encoding an antigen or a variant thereof, wherein the T cells genetically modified to express a CAR are targeted to the antigen. In one particularly preferred embodiment, the polynucleotides administered according to the present disclosure are RNA.

### Background

The immune system plays an important role in cancer, autoimmunity, allergies as well as in pathogen-associated diseases. T cells play a central role in cell-mediated immunity in humans and animals. The recognition and binding of a particular antigen by T cells is mediated by the T cell receptors (TCRs) expressed on the surface of T cells. The TCR of a T cell is able to interact with immunogenic peptides (epitopes) bound to major histocompatibility complex (MHC) molecules and presented on the surface of target cells. Specific binding of the TCR triggers a signal cascade inside the T cell leading to proliferation and differentiation into a maturated effector T cell.

The diversity of TCRs is obtained by genetic rearrangement of different discontinuous segments of genes which code for the different structural regions of TCRs. TCRs are composed of one α-chain and one β-chain or of one γ-chain and one δ-chain. The TCR α/β chains are composed of an N-terminal highly polymorphic variable region involved in antigen recognition and an invariant constant region. On the genetic level, these chains are separated into several regions, a variable (V) region, a diversity (D) region (only β- and δ-chain), a joining (J) region and a constant (C) region. During the differentiation of T cells, specific T cell receptor genes are created by rearranging one V, one D (only β- and δ-chain), one J and one C region gene. The diversity of the TCRs is further amplified by imprecise V-(D)-J rearrangement wherein random nucleotides are introduced and/or deleted at the recombination sites. Since the rearrangement of the TCR gene loci occurs in the genome during maturation of T cells, each mature T cell only expresses one specific α/β TCR or γ/δ TCR. The TCR is a part of a complex signaling machinery, which includes the heterodimeric complex of the TCR α- and β-chains, the co-receptor CD4 or CD8 and the CD3 signal transduction module. While the CD3 chains transfer the activation signal inside the cell, the TCR α/β heterodimer is solely responsible for antigen recognition.

Adoptive cell transfer (ACT) based immunotherapy can be broadly defined as a form of passive immunization with previously sensitized T cells that are transferred to non-immune recipients or to the autologous host after ex *vivo* expansion from low precursor frequencies to clinically relevant cell numbers. Cell types that have been used for ACT experiments are lymphokine-activated killer (LAK) cells (Mule, J.J. et al. (1984) Science 225, 1487-1489; Rosenberg, S.A. et al. (1985) N. Engl. J. Med. 313, 1485-1492), tumor-infiltrating lymphocytes (TILs) (Rosenberg, S.A. et al. (1994) J. Natl. Cancer Inst. 86, 1159-1166), donor lymphocytes after hematopoietic stem cell transplantation (HSCT) as well as tumor-specific T cell lines or clones (Dudley, M.E. et al. (2001) J. Immunother. 24, 363-373; Yee, C. et al. (2002) Proc. Natl. Acad. Sci. U. S. A 99, 16168-16173). Adoptive T cell transfer was shown to have therapeutic activity against human viral infections such as CMV. While CMV infection and reactivation of endogenous latent viruses is controlled by the immune system in healthy individuals, it results in significant morbidity and mortality in immune compromised individuals such as transplant recipients or AIDS patients. Riddell and co-workers demonstrated the reconstitution of viral immunity by adoptive T cell therapy in immune suppressed patients after transfer of CD8+ CMV-specific T cell clones derived from HLA-matched CMV-seropositive transplant donors (Riddell, S.R. (1992) Science 257, 238-241). As an alternative approach polyclonal donor-derived CMV- or EBV-specific T cell populations were transferred to transplant recipients resulting in increased persistence of transferred T cells (Rooney, C.M. et al. (1998) Blood 92, 1549-1555; Peggs, K.S. et al. (2003) Lancet 362, 1375-1377). For adoptive immunotherapy of melanoma, Rosenberg and co-workers established an ACT approach relying on the infusion of *in vitro* expanded autologous tumor-infiltrating lymphocytes (TILs) isolated from excised tumors in combination with a non-myeloablative lymphodepleting chemotherapy and high-dose IL2. A recently published clinical study resulted in an objective response rate of -50% of treated patients suffering from metastatic melanoma (Dudley, M.E. et al. (2005) J. Clin. Oncol. 23: 2346-2357).

An alternative approach is the adoptive transfer of autologous T cells reprogrammed to express a tumor-reactive immunoreceptor of defined specificity during short-time ex *vivo* culture followed by reinfusion into the patient (Kershaw M.H. et al. (2013) Nature Reviews Cancer 13 (8):525-41). This strategy makes ACT applicable to a variety of common malignancies even if tumor-reactive T cells are absent in the patient. Since the antigenic specificity of T cells is rested entirely on the heterodimeric complex of the TCR α- and β-chain, the transfer of cloned TCR genes into T cells offers the potential to redirect them towards any antigen of interest. Therefore, TCR gene therapy provides an attractive strategy to develop antigen-specific immunotherapy with autologous lymphocytes as treatment option. Major advantages of TCR gene transfer are the creation of therapeutic quantities of antigen-specific T cells within a few days and the possibility to introduce specificities that are not present in the endogenous TCR repertoire of the patient.

Several groups demonstrated, that TCR gene transfer is an attractive strategy to redirect antigen-specificity of primary T cells (Morgan, R.A. et al. (2003) J. Immunol. 171, 3287-3295; Cooper, L.J. et al. (2000) J. Virol. 74, 8207-8212; Fujio, K. et al. (2000) J. Immunol. 165, 528-532; Kessels, H.W. et al. (2001) Nat. Immunol. 2, 957-961; Dembic, Z. et al. (1986) Nature 320, 232-238). Feasibility of TCR gene therapy in humans was initially demonstrated in clinical trials for the treatment of malignant melanoma by Rosenberg and his group. The adoptive transfer of autologous lymphocytes retrovirally transduced with melanomalmelanocyte antigen-specific TCRs resulted in cancer regression in up to 30% of treated melanoma patients (Morgan, R.A. et al. (2006) Science 314, 126-129; Johnson, L.A. et al. (2009) Blood 114, 535-546). In the meantime clinical testing of TCR gene therapy was extended also to cancers other than melanoma targeting many different tumor antigens (Park, T.S. et al., (2011) Trends Biotechnol. 29, 550-557).

The use of genetic engineering approaches to insert antigen-targeted receptors of defined specificity into T cells has greatly extended the potential capabilities of ACT. Chimeric antigen receptors (CARs) are a type of antigen-targeted receptor composed of intracellular T cell signaling domains fused to extracellular antigen-binding moieties, most commonly single-chain variable fragments (scFvs) from monoclonal antibodies. CARs directly recognize cell surface antigens, independent of MHC-mediated presentation, permitting the use of a single receptor construct specific for any given antigen in all patients. Initial CARs fused antigen-recognition domains to the CD3ζ activation chain of the T cell receptor (TCR) complex. Subsequent CAR iterations have included secondary costimulatory signals in tandem with CD3ζ, including intracellular domains from CD28 or a variety of TNF receptor family molecules such as 4-1 BB (CD137) and OX40 (CD134). Further, third generation receptors include two costimulatory signals in addition to CD3ζ, most commonly from CD28 and 4-1BB. Second and third generation CARs dramatically improved antitumor efficacy, in some cases inducing complete remissions in patients with advanced cancer.

It is generally thought that the number of transferred T cells is correlated with therapeutic responses. However, the number of cells which can be administered to a patient for adoptive T cell transfer is limited and the generation of a large amount of T cells for adoptive T cell transfer still remains a challenge. A substantial increase in cell persistence could be achieved when patients received a lymphodepleting preparative regimen before infusion of either TILs or receptor-engineered T cells. However, the transfer of a large amount of engineered T cells into an empty host also poses the risk of severe adverse events in case that the targeted antigen is unexpectedly expressed in a relevant normal tissue. Therefore, it would be desirable to transfer a limited amount of engineered T cells that can be expanded in the patient after they have proven to be safe.

The present inventors found that it is possible to expand CAR-T cells in a subject by administering RNA encoding IL2 optionally in combination with RNA encoding a further cytokine such as IL7 or IL21 and optionally using RNA-vaccination to provide antigen for CAR-T cell stimulation. The methods of the invention allow to only provide small amounts of CAR-engineered T cells to a patient and then expand the T cells *in vivo.*

### Summary

The present invention generally embraces the treatment of diseases by targeting cells expressing an antigen on the cell surface such as diseased cells expressing an antigen on the cell surface, in particular cancer cells expressing a tumor antigen on the cell surface. The methods provide for the selective eradication of cells that express on their surface an antigen, thereby minimizing adverse effects to normal cells not expressing the antigen. T cells genetically modified to express a chimeric antigen receptor (CAR) targeting the cells through binding to the antigen are provided in a subject such as by administration of the T cells. IL2 or nucleic acid coding therefor is administered. In one embodiment, a further cytokine such as IL7 or IL21 or nucleic acid coding therefor is administered. In one embodiment, the antigen or a variant thereof or nucleic acid coding therefor is administered to provide (optionally following expression of the nucleic acid by appropriate target cells) antigen for T cell stimulation, priming and/or expansion. T cells stimulated, primed and/or expanded in the patient are able to recognize cells expressing an antigen on the cell surface such as diseased cells, resulting in the eradication of diseased cells. The present approach can be considered to involve passive and active immunization. Treatment involving administration of T cells genetically modified to express a CAR can be considered as a form of passive immunization. Treatment involving administration of antigen or a variant thereof, thereby stimulating a T cell-mediated immune response to a target cell population or tissue, can be considered as a form of active immunization.

The immune response according to the present disclosure is to a target cell population or target tissue expressing an antigen in a mammal and the T cells genetically modified to express a chimeric antigen receptor (CAR) are targeted to the antigen. The present methods optionally also involve administration of the antigen or a variant thereof. In one embodiment, the immune response is a T cell-mediated immune response. In one embodiment, the immune response is an anti-tumor immune response and the target cell population or target tissue is tumor cells or tumor tissue.

The methods and agents described herein are particularly effective if RNA encoding IL2 attached to a pharmacokinetic modifying group (hereafter referred to as "extended-pharmacokinetic (PK) IL2") optionally in combination with RNA encoding a further cytokine such as IL7 or IL21 attached to a pharmacokinetic modifying group (hereafter referred to as "extended-pharmacokinetic (PK) cytokine") is administered. The methods and agents described herein are particularly effective if the RNA encoding extended-PK IL2 and/or the RNA encoding extended-PK cytokine is targeted to the liver for systemic availability. Liver cells can be efficiently transfected and are able to produce large amounts of protein. Antigen-encoding RNA is preferably targeted to secondary lymphoid organs.

In one aspect, the invention provides a method for inducing an immune response in a subject comprising:
a. providing to the subject T cells genetically modified to express a chimeric antigen receptor (CAR) and
b. administering to the subject IL2 or a polynucleotide encoding IL2.

In one embodiment, the method comprises administering IL2 or a polynucleotide encoding IL2 and a further cytokine or a polynucleotide encoding a further cytokine. In one embodiment, the further cytokine is selected from the group consisting of IL7 and IL21. In one embodiment, the method comprises administering IL2 or a polynucleotide encoding IL2 and IL7 or a polynucleotide encoding IL7. In one embodiment, the method comprises administering IL2 or a polynucleotide encoding IL2 and IL21 or a polynucleotide encoding IL21.

In one embodiment, the polynucleotide encoding IL2 is RNA and optionally the polynucleotide encoding a further cytokine is RNA.

In one embodiment, the T cells genetically modified to express a CAR are provided to the subject by administering the T cells genetically modified to express a CAR or by generating the T cells genetically modified to express a CAR in the subject.

In one embodiment, the method further comprises administering to the subject an antigen or a variant thereof, or a polynucleotide encoding the antigen or variant, wherein the T cells genetically modified to express a CAR are targeted to the antigen and the immune response is an immune response to a target cell population or target tissue expressing the antigen. In one embodiment, the polynucleotide encoding the antigen or variant is RNA.

In one aspect, the invention provides a method for inducing an immune response in a subject comprising:
a. providing to the subject T cells genetically modified to express a chimeric antigen receptor (CAR) and
b. administering to the subject RNA encoding IL2.

In one embodiment, the method comprises administering RNA encoding IL2 and RNA encoding a further cytokine. In one embodiment, the further cytokine is selected from the group consisting of IL7 and IL21. In one embodiment, the method comprises administering RNA encoding IL2 and RNA encoding IL7. In one embodiment, the method comprises administering RNA encoding IL2 and RNA encoding IL21.

In one embodiment, the T cells genetically modified to express a CAR are provided to the subject by administering the T cells genetically modified to express a CAR or by generating the T cells genetically modified to express a CAR in the subject.

In one embodiment, the method further comprises administering to the subject RNA encoding an antigen or a variant thereof, wherein the T cells genetically modified to express a CAR are targeted to the antigen and the immune response is an immune response to a target cell population or target tissue expressing the antigen.

In one embodiment of all aspects, the immune response is a T cell-mediated immune response.

In one aspect, the invention provides a method for treating a subject having a disease, disorder or condition associated with expression or elevated expression of an antigen comprising:
a. providing to the subject T cells genetically modified to express a chimeric antigen receptor (CAR) targeted to the antigen and
b. administering to the subject IL2 or a polynucleotide encoding IL2.

In one embodiment, the method comprises administering IL2 or a polynucleotide encoding IL2 and a further cytokine or a polynucleotide encoding a further cytokine. In one embodiment, the further cytokine is selected from the group consisting of IL7 and IL21. In one embodiment, the method comprises administering IL2 or a polynucleotide encoding IL2 and IL7 or a polynucleotide encoding IL7. In one embodiment, the method comprises administering IL2 or a polynucleotide encoding IL2 and IL21 or a polynucleotide encoding IL21.

In one embodiment, the polynucleotide encoding IL2 is RNA and optionally the polynucleotide encoding a further cytokine is RNA.

In one embodiment, the T cells genetically modified to express a CAR are provided to the subject by administering the T cells genetically modified to express a CAR or by generating the T cells genetically modified to express a CAR in the subject.

In one embodiment, the method further comprises administering to the subject the antigen or a variant thereof, or a polynucleotide encoding the antigen or variant. In one embodiment, the polynucleotide encoding the antigen or variant is RNA.

In one aspect, the invention provides a method for treating a subject having a disease, disorder or condition associated with expression or elevated expression of an antigen comprising:
a. providing to the subject T cells genetically modified to express a chimeric antigen receptor (CAR) targeted to the antigen and
b. administering to the subject RNA encoding IL2.

In one embodiment, the method comprises administering RNA encoding IL2 and RNA encoding a further cytokine. In one embodiment, the further cytokine is selected from the group consisting of IL7 and IL21. In one embodiment, the method comprises administering RNA encoding IL2 and RNA encoding IL7. In one embodiment, the method comprises administering RNA encoding IL2 and RNA encoding IL21.

In one embodiment, the T cells genetically modified to express a CAR are provided to the subject by administering the T cells genetically modified to express a CAR or by generating the T cells genetically modified to express a CAR in the subject.

In one embodiment, the method further comprises administering to the subject RNA encoding the antigen or a variant thereof.

In one embodiment of all aspects, the disease, disorder or condition is cancer and the antigen is a tumor-associated antigen.

In one embodiment of all aspects, IL2 is extended pharmacokinetic (PK) IL2. In one embodiment, the extended-PK IL2 comprises a fusion protein. In one embodiment, the fusion protein comprises an IL2 moiety and a moiety selected from the group consisting of serum albumin, an immunoglobulin fragment, transferrin, Fn3, and variants thereof.

In one embodiment of all aspects, the further cytokine, in particular IL7 or IL21, is extended pharmacokinetic (PK) cytokine, in particular extended-PK IL7 or extended-PK IL21. In one embodiment, the extended-PK cytokine, in particular extended-PK IL7 or extended-PK IL21, comprises a fusion protein. In one embodiment, the fusion protein comprises a moiety of the further cytokine, in particular an IL7 moiety or an IL21 moiety, and a moiety selected from the group consisting of serum albumin, an immunoglobulin fragment, transferrin, Fn3, and variants thereof.

In one embodiment, the serum albumin comprises mouse serum albumin or human serum albumin. In one embodiment, the immunoglobulin fragment comprises an immunoglobulin Fc domain.

In one embodiment of all aspects, the method is a method for treating or preventing cancer in a subject, wherein the antigen is a tumor-associated antigen.

In one aspect, the invention provides a medical preparation comprising:
a. T cells genetically modified to express a chimeric antigen receptor (CAR) and
b. IL2 or a polynucleotide encoding IL2.

In one embodiment, the medical preparation comprises IL2 or a polynucleotide encoding IL2 and a further cytokine or a polynucleotide encoding a further cytokine. In one embodiment, the further cytokine is selected from the group consisting of IL7 and IL21. In one embodiment, the medical preparation comprises IL2 or a polynucleotide encoding IL2 and IL7 or a polynucleotide encoding IL7. In one embodiment, the medical preparation comprises IL2 or a polynucleotide encoding IL2 and IL21 or a polynucleotide encoding IL21.

In one embodiment, the polynucleotide encoding IL2 is RNA and optionally the polynucleotide encoding a further cytokine is RNA.

In one embodiment, the medical preparation further comprises an antigen or a variant thereof, or a polynucleotide encoding the antigen or variant, wherein the T cells genetically modified to express a CAR are targeted to the antigen. In one embodiment, the polynucleotide encoding the antigen or variant is RNA.

In one embodiment, the medical preparation is a kit.

In one embodiment, the medical preparation comprises the T cells genetically modified to express a CAR, the IL2 or the polynucleotide encoding IL2, optionally the further cytokine or the polynucleotide encoding a further cytokine and optionally the antigen or a variant thereof, or the polynucleotide encoding the antigen or variant in separate containers.

In one embodiment, the medical preparation further comprises instructions for use of the medical preparation for treating or preventing cancer wherein the antigen is a tumor-associated antigen.

In one embodiment, the medical preparation is a pharmaceutical composition.

In one embodiment, the pharmaceutical composition further comprises one or more pharmaceutically acceptable carriers, diluents and/or excipients.

In one aspect, the invention provides a medical preparation comprising:
a. T cells genetically modified to express a chimeric antigen receptor (CAR) and
b. RNA encoding IL2.

In one embodiment, the medical preparation comprises RNA encoding IL2 and RNA encoding a further cytokine. In one embodiment, the further cytokine is selected from the group consisting of IL7 and IL21. In one embodiment, the medical preparation comprises RNA encoding IL2 and RNA encoding IL7. In one embodiment, the medical preparation comprises RNA encoding IL2 and RNA encoding IL21.

In one embodiment, the medical preparation further comprises RNA encoding an antigen or a variant thereof, wherein the T cells genetically modified to express a CAR are targeted to the antigen.

In one embodiment, the medical preparation is a kit.

In one embodiment, the medical preparation comprises the T cells genetically modified to express a CAR, the RNA encoding IL2, optionally the RNA encoding a further cytokine, and optionally the RNA encoding an antigen or a variant thereof in separate containers.

In one embodiment, the medical preparation further comprises instructions for use of the medical preparation for treating or preventing cancer wherein the antigen is a tumor-associated antigen.

In one embodiment, the medical preparation is a pharmaceutical composition.

In one embodiment, the pharmaceutical composition further comprises one or more pharmaceutically acceptable carriers, diluents and/or excipients.

In one embodiment of all aspects, IL2 is extended pharmacokinetic (PK) IL2. In one embodiment, the extended-PK IL2 comprises a fusion protein. In one embodiment, the fusion protein comprises an IL2 moiety and a moiety selected from the group consisting of serum albumin, an immunoglobulin fragment, transferrin, Fn3, and variants thereof.

In one embodiment of all aspects, the further cytokine is extended pharmacokinetic (PK) cytokine. In one embodiment, the extended-PK cytokine comprises a fusion protein. In one embodiment, the fusion protein comprises a cytokine moiety and a moiety selected from the group consisting of serum albumin, an immunoglobulin fragment, transferrin, Fn3, and variants thereof.

In one embodiment, the serum albumin comprises mouse serum albumin or human serum albumin.

In one embodiment, the immunoglobulin fragment comprises an immunoglobulin Fc domain.

In one aspect, the invention provides the medical preparation described herein for pharmaceutical use. In one embodiment, the pharmaceutical use comprises a therapeutic or prophylactic treatment of a disease or disorder.

In one aspect, the invention provides the medical preparation described herein for use in a method for treating or preventing cancer in a subject, wherein the antigen is a tumor-associated antigen.

In one aspect, the invention provides the agents and compositions described herein for use in the methods described herein.

In one aspect, the invention provides T cells genetically modified to express a chimeric antigen receptor (CAR) targeted to an antigen for use in the methods described herein.

In one aspect, the invention provides IL2 or a polynucleotide encoding IL2 for use in the methods described herein.

In one aspect, the invention provides a cytokine other than IL2 such as IL7 or IL21 or a polynucleotide coding therefor for use in the methods described herein.

In one aspect, the invention provides an antigen or a variant thereof or a nucleic acid encoding an antigen or a variant thereof for use in the methods described herein.

In one embodiment of the medical preparation, the RNA is present in a form selected from a liquid form, a solid form, or a combination thereof. In one embodiment, the solid form is a frozen form or a dehydrated form. In one embodiment, the dehydrated form is a freeze-dried or spray-dried form.

In one embodiment, the cancer described herein is selected from the group consisting of melanoma, leukemia, lymphoma, lung cancer, breast cancer, prostate cancer, ovarian cancer, colon cancer, mesothelioma, renal cell carcinoma, and brain cancer.

### Brief description of the drawings

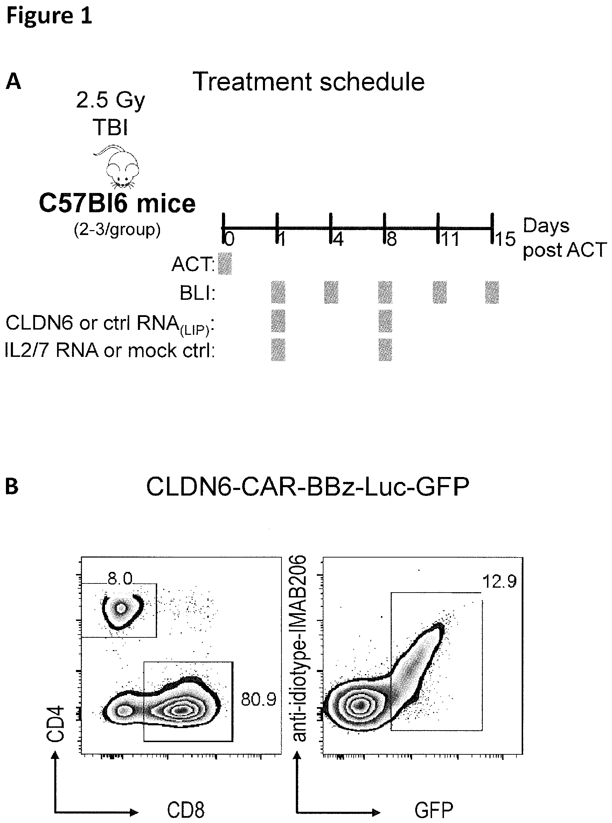

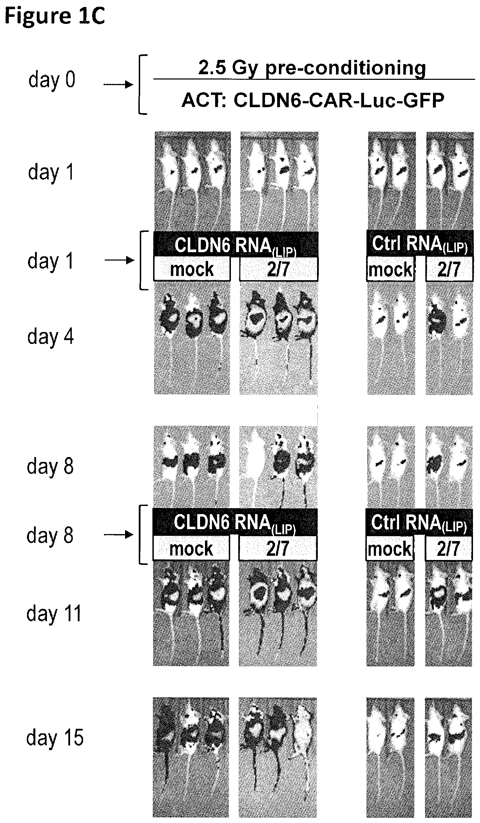

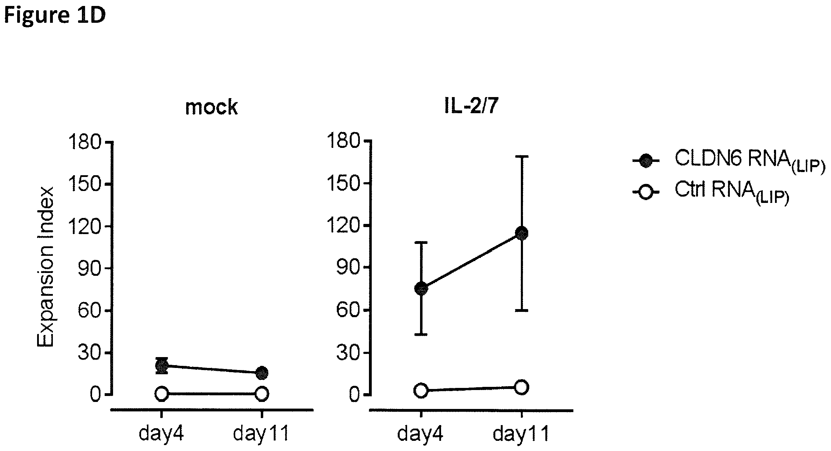

**Figure 1****: mAlb-mIL-2 and mlL-7-mAlb enhance *in-situ* repetitive antigen-specific expansion of genetically engineered CAR T cells *in vivo* in pre-conditioned mice.** (A) 2.5 Gy irradiated (XRAD320) C57BL/6BrdCrHsd-*Tyr^{c}* mice (n = 2-3/group) were *i.v*. engrafted with 5 × 10⁶ CLDN6-CAR-BBz-Luc-GFP transduced C57BI/6-Thy1.1⁺ T cells. One day later, mice were treated with hCLDN6 or Oval (ctrl RNA) encoding mRNA lipoplex vaccination (20 µg, *i.v.*) followed by *i.p*. administration of nucleoside-modified-formulated RNA encoding **mAlb-mIL-2** and mIL-7-mAlb (1 µg/ mRNAI mouse). Buffer was used as mock control. After additional 7 days, the treatment was repeated. Bioluminescence imaging (BLI) was performed to monitor expansion and persistence on day 1 (baseline) up to day 15 after ACT. (B) Transgene expression of adoptively transferred murine CAR transduced T cells. Cells were stained with fluorochrome-conjugated antibodies directed against CD8 and CD4 as well as with an idiotype-specific antibody directed against the scFv part of the CLDN6-CAR (anti-IMAB206) and analyzed by flow cytometry. Left, cells were gated on single lymphocytes; right, cells were gated on CD8+ T cells. (C) Bioluminescence imaging of mice in lateral position at various time points after ACT and treatment with antigen RNA_{(LlP)} in combination with albumin-cytokine encoding mRNAs as indicated. Off-color images represent light intensity (black, least intense; white up to dark-grey, most intense) which was superimposed over the greyscale reference images. (D) The calculated expansion index (mean±s.d.) of total flux after 4 and 11 days post ACT compared to baseline at day 1; ACT: adoptive T cell transfer, TBI: total body irradiation, BLI: bioluminescence imaging, Luc: effective firefly luciferase, mAlb: murine serum albumin, mIL-2: murine Interleukin-2, mIL-7: murine Interleukin-7.

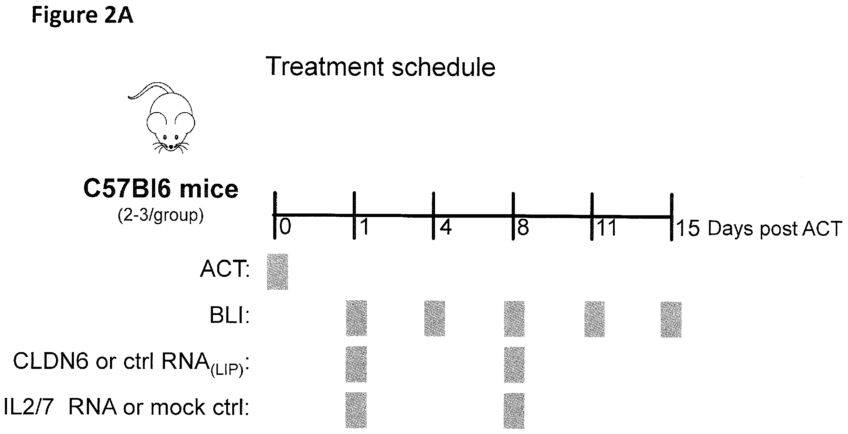

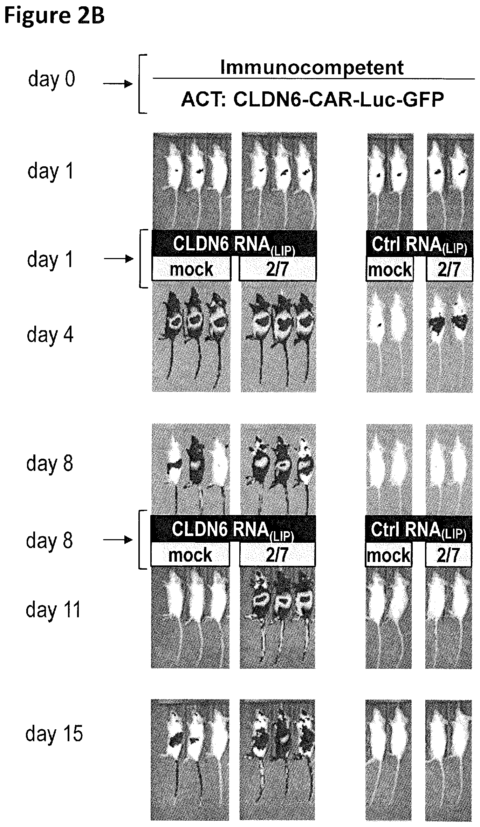

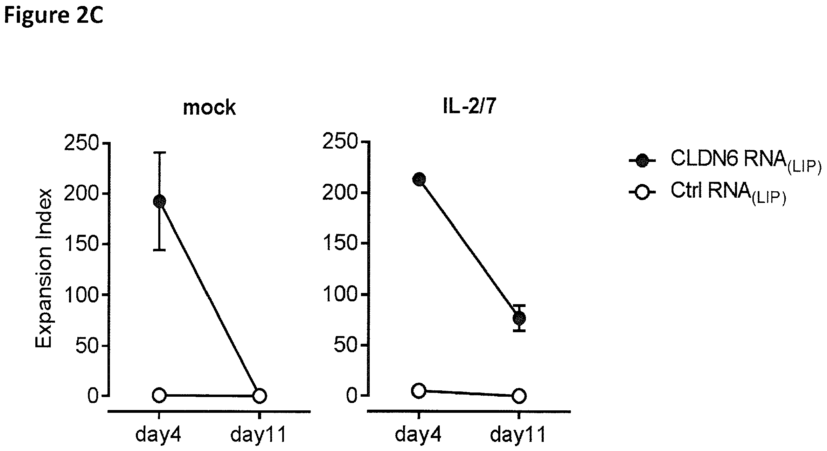

**Figure 2****: mAlb-mIL-2 and mIL-7-mAlb prolong persistence of antigen-specific expanded CAR T cells *in vivo* even in immunocompetent mice.** (A) Non-irradiated C57BL/6BrdCrHsd-*Tyr^{c}* mice (n = 2-3/group) received the same dose of CAR-transduced T cells and were treated as in Figure legend 1 A-B described. (B) Bioluminescence imaging of mice in lateral position at various time points after ACT and treatment with antigen RNA_{(LlP)} in combination with albumin-cytokine encoding mRNAs as indicated. Off-color images represent light intensity (black, least intense; white up to dark-grey, most intense) which was superimposed over the greyscale reference images. (C) The expansion index (mean±s.d.) of total flux after 4 and 11 days post ACT compared to baseline at day 1; ACT: adoptive T cell transfer, BLI: bioluminescence imaging, Luc: effective firefly luciferase, mAlb: murine serum albumin, mIL-2: murine Interleukin-2, mIL-7: murine Interleukin-7.

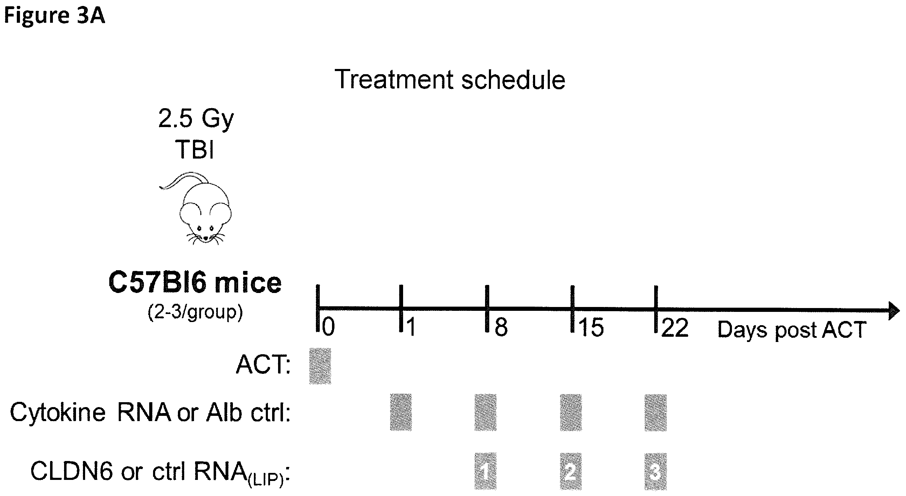

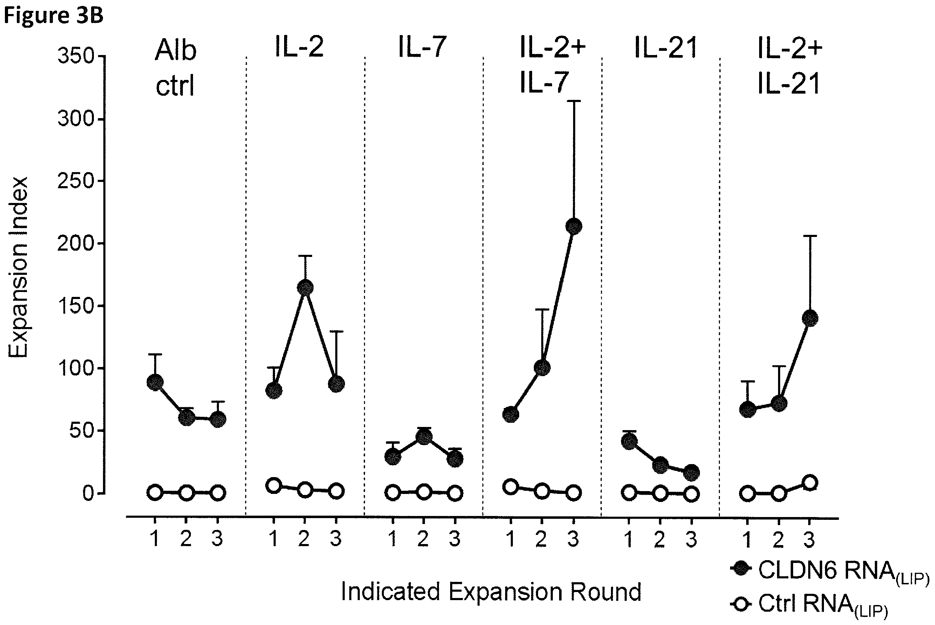

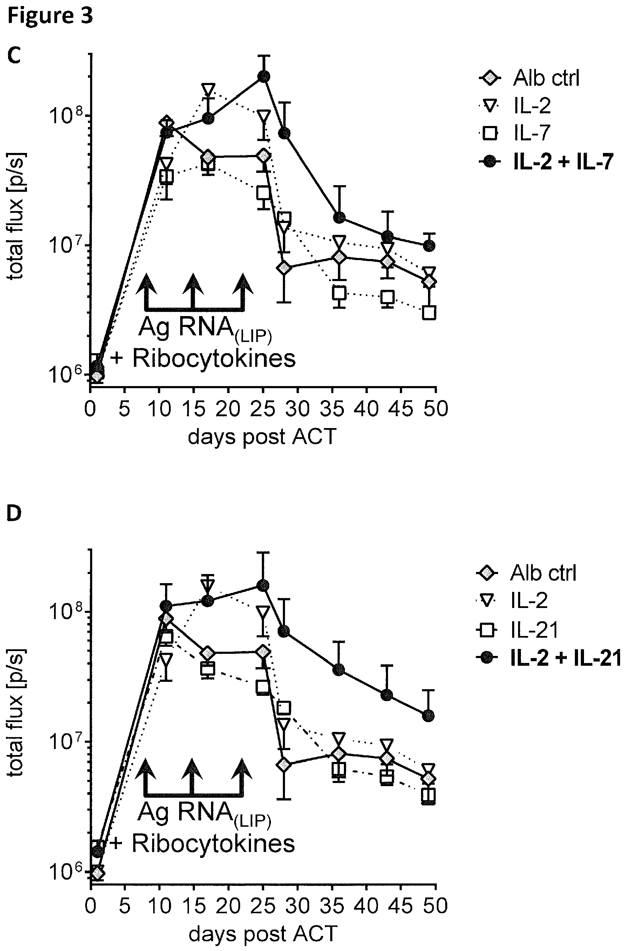

**Figure 3****: The presence of mAlb-mIL-2 in combination with either mIL-7-mAlb or mIL-21-mAlb resulted in an accumulation of in-situ repetitive antigen-specific expansion and prolonged persistence of genetically engineered CAR T cells *in vivo.*** (A) 2.5 Gy irradiated C57BL/6BrdCrHsd-*Tyr^{c}* mice (n = 2-3/group) received the same dose of CAR-transduced T cells and hCLDN6 or Oval (ctrl RNA) encoding mRNA lipoplex vaccination in combination with different nucleoside-modified-formulated cytokine (1µg per individual used mRNA/ animal) as described in Figure 1A. (B) Relative increase in bioluminescence of treated mice was quantified and calculated during 3 vaccination rounds (imaging was usually performed 2-3 days after indicated antigen-RNA_{(LlP)} and cytokine RNA treatment round). Expansion index was calculated as follows: total flux [p/s] of respective expansion round / total flux [p/s] of baseline at day one after ACT (mean±s.e.m.). (C-D) Quantification of bioluminescence during and after the expansion rounds with CLDN6-RNA_{(LIP)} in the presence of indicated nucleoside-modified-formulated cytokine RNAs (mean+/- s.e.m.). Arrows indicate the hCLDN6 RNA_{(LIP)} vaccination and nucleoside-modified-formulated cytokine (ribocytokine) treatment. ACT: adoptive T cell transfer, TBI: total body irradiation, BLI: bioluminescence imaging, Luc: effective firefly luciferase, mAlb: murine serum albumin, mIL-2: murine Interleukin-2, mIL-7: murine Interleukin-7.

### Detailed description

Although the present disclosure is described in detail below, it is to be understood that this disclosure is not limited to the particular methodologies, protocols and reagents described herein as these may vary. It is also to be understood that the terminology used herein is for the purpose of describing particular embodiments only, and is not intended to limit the scope of the present disclosure which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The practice of the present disclosure will employ, unless otherwise indicated, conventional methods of chemistry, biochemistry, cell biology, immunology, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

In the following, the elements of the present disclosure will be described. These elements are listed with specific embodiments, however, it should be understood that they may be combined in any manner and in any number to create additional embodiments. The variously described examples and embodiments should not be construed to limit the present disclosure to only the explicitly described embodiments, This description should be understood to disclose and encompass embodiments which combine the explicitly described embodiments with any number of the disclosed elements. Furthermore, any permutations and combinations of all described elements should be considered disclosed by this description unless the context indicates otherwise.

The term "about" means approximately or nearly, and in the context of a numerical value or range set forth herein in one embodiment means ± 20%, ± 10%, ± 5%, or ± 3% of the numerical value or range recited or claimed.

The terms "a" and "an" and "the" and similar reference used in the context of describing the disclosure (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it was individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the disclosure and does not pose a limitation on the scope of the claims. No language in the specification should be construed as indicating any non-claimed element essential to the practice of the disclosure.

Unless expressly specified otherwise, the term "comprising" is used in the context of the present document to indicate that further members may optionally be present in addition to the members of the list introduced by "comprising". It is, however, contemplated as a specific embodiment of the present disclosure that the term "comprising" encompasses the possibility of no further members being present, i.e., for the purpose of this embodiment "comprising" is to be understood as having the meaning of "consisting of".

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the present disclosure was not entitled to antedate such disclosure.

In the following, definitions will be provided which apply to all aspects of the present disclosure. The following terms have the following meanings unless otherwise indicated. Any undefined terms have their art recognized meanings.

According to the disclosure, the term "peptide" comprises oligo- and polypeptides and refers to substances which comprise about two or more, about 3 or more, about 4 or more, about 6 or more, about 8 or more, about 10 or more, about 13 or more, about 16 or more, about 20 or more, and up to about 50, about 100 or about 150, consecutive amino acids linked to one another via peptide bonds. The term "protein" or "polypeptide" refers to large peptides, in particular peptides having at least about 151 amino acids, but the terms "peptide", "protein" and "polypeptide" are used herein usually as synonyms.

A "therapeutic protein" has a positive or advantageous effect on a condition or disease state of a subject when provided to the subject in a therapeutically effective amount. In one embodiment, a therapeutic protein has curative or palliative properties and may be administered to ameliorate, relieve, alleviate, reverse, delay onset of or lessen the severity of one or more symptoms of a disease or disorder. A therapeutic protein may have prophylactic properties and may be used to delay the onset of a disease or to lessen the severity of such disease or pathological condition. The term "therapeutic protein" includes entire proteins or peptides, and can also refer to therapeutically active fragments thereof. It can also include therapeutically active variants of a protein. Examples of therapeutically active proteins include, but are not limited to, cytokines.

"Fragment", with reference to an amino acid sequence (peptide or protein), relates to a part of an amino acid sequence, i.e. a sequence which represents the amino acid sequence shortened at the N-terminus and/or C-terminus. A fragment shortened at the C-terminus (N-terminal fragment) is obtainable e.g. by translation of a truncated open reading frame that lacks the 3'-end of the open reading frame. A fragment shortened at the N-terminus (C-terminal fragment) is obtainable e.g. by translation of a truncated open reading frame that lacks the 5'-end of the open reading frame, as long as the truncated open reading frame comprises a start codon that serves to initiate translation. A fragment of an amino acid sequence comprises e.g. at least 50 %, at least 60 %, at least 70 %, at least 80%, at least 90% of the amino acid residues from an amino acid sequence. A fragment of an amino acid sequence preferably comprises at least 6, in particular at least 8, at least 12, at least 15, at least 20, at least 30, at least 50, or at least 100 consecutive amino acids from an amino acid sequence.

For the purposes of the present disclosure, "variants" of an amino acid sequence (peptide or protein) comprise amino acid insertion variants, amino acid addition variants, amino acid deletion variants and/or amino acid substitution variants. The term "variant" includes, in particular, fragments of an amino acid sequence.

Amino acid insertion variants comprise insertions of single or two or more amino acids in a particular amino acid sequence. In the case of amino acid sequence variants having an insertion, one or more amino acid residues are inserted into a particular site in an amino acid sequence, although random insertion with appropriate screening of the resulting product is also possible. Amino acid addition variants comprise amino- and/or carboxy-terminal fusions of one or more amino acids, such as 1, 2, 3, 5, 10, 20, 30, 50, or more amino acids. Amino acid deletion variants are characterized by the removal of one or more amino acids from the sequence, such as by removal of 1, 2, 3, 5, 10, 20, 30, 50, or more amino acids. The deletions may be in any position of the protein. Amino acid deletion variants that comprise the deletion at the N-terminal and/or C-terminal end of the protein are also called N-terminal and/or C-terminal truncation variants. Amino acid substitution variants are characterized by at least one residue in the sequence being removed and another residue being inserted in its place. Preference is given to the modifications being in positions in the amino acid sequence which are not conserved between homologous proteins or peptides and/or to replacing amino acids with other ones having similar properties. Preferably, amino acid changes in peptide and protein variants are conservative amino acid changes, i.e., substitutions of similarly charged or uncharged amino acids. A conservative amino acid change involves substitution of one of a family of amino acids which are related in their side chains. Naturally occurring amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine) amino acids. Phenylalanine, tryptophan, and tyrosine are sometimes classified jointly as aromatic amino acids.

Preferably the degree of similarity, preferably identity between a given amino acid sequence and an amino acid sequence which is a variant of said given amino acid sequence will be at least about 60%, 65%, 70%, 80%, 81%, 82%, 83%, 84%, 85%, 86%, 87%, 88%, 89%, 90%, 91%, 92%, 93%, 94%, 95%, 96%, 97%, 98%, or 99%. The degree of similarity or identity is given preferably for an amino acid region which is at least about 10%, at least about 20%, at least about 30%, at least about 40%, at least about 50%, at least about 60%, at least about 70%, at least about 80%, at least about 90% or about 100% of the entire length of the reference amino acid sequence. For example, if the reference amino acid sequence consists of 200 amino acids, the degree of similarity or identity is given preferably for at least about 20, at least about 40, at least about 60, at least about 80, at least about 100, at least about 120, at least about 140, at least about 160, at least about 180, or about 200 amino acids, preferably continuous amino acids. In preferred embodiments, the degree of similarity or identity is given for the entire length of the reference amino acid sequence. The alignment for determining sequence similarity, preferably sequence identity can be done with art known tools, preferably using the best sequence alignment, for example, using Align, using standard settings, preferably EMBOSS::needle, Matrix: Blosum62, Gap Open 10.0, Gap Extend 0.5.

"Sequence similarity" indicates the percentage of amino acids that either are identical or that represent conservative amino acid substitutions. "Sequence identity" between two amino acid sequences indicates the percentage of amino acids that are identical between the sequences.

The term "percentage identity" is intended to denote a percentage of amino acid residues which are identical between the two sequences to be compared, obtained after the best alignment, this percentage being purely statistical and the differences between the two sequences being distributed randomly and over their entire length. Sequence comparisons between two amino acid sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Neddleman and Wunsch, 1970, J. Mol. Biol. 48, 443, by means of the similarity search method of Pearson and Lipman, 1988, Proc. Natl Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

The percentage identity is calculated by determining the number of identical positions between the two sequences being compared, dividing this number by the number of positions compared and multiplying the result obtained by 100 so as to obtain the percentage identity between these two sequences.

Homologous amino acid sequences exhibit according to the disclosure at least 40%, in particular at least 50%, at least 60%, at least 70%, at least 80%, at least 90% and preferably at least 95%, at least 98 or at least 99% identity of the amino acid residues.

The amino acid sequence variants described herein may readily be prepared by the skilled person, for example, by recombinant DNA manipulation. The manipulation of DNA sequences for preparing peptides or proteins having substitutions, additions, insertions or deletions, is described in detail in Sambrook et al. (1989), for example. Furthermore, the peptides and amino acid variants described herein may be readily prepared with the aid of known peptide synthesis techniques such as, for example, by solid phase synthesis and similar methods.

In one embodiment, a fragment or variant of an amino acid sequence (peptide or protein) is preferably a "functional fragment" or "functional variant". The term "functional fragment" or "functional variant" of an amino acid sequence relates to any fragment or variant exhibiting one or more functional properties identical or similar to those of the amino acid sequence from which it is derived, i.e., it is functionally equivalent. With respect to cytokines, one particular function is one or more immunomodulatory activities displayed by the amino acid sequence from which the fragment or variant is derived and/or binding to the receptor(s) the amino acid sequence from which the fragment or variant is derived binds to.

An amino acid sequence (peptide or protein) "derived from" a designated amino acid sequence (peptide or protein) refers to the origin of the first amino acid sequence. Preferably, the amino acid sequence which is derived from a particular amino acid sequence has an amino acid sequence that is identical, essentially identical or homologous to that particular sequence or a fragment thereof. Amino acid sequences derived from a particular amino acid sequence may be variants of that particular sequence or a fragment thereof. For example, it will be understood by one of ordinary skill in the art that the antigens and cytokines (e.g., IL2, IL7 or IL21) suitable for use herein may be altered such that they vary in sequence from the naturally occurring or native sequences from which they were derived, while retaining the desirable activity of the native sequences.

### T cells

T cells belong to a group of white blood cells known as lymphocytes, and play a central role in cell-mediated immunity. They can be distinguished from other lymphocyte types, such as B cells and natural killer cells by the presence of a special receptor on their cell surface called T cell receptors (TCR). The thymus is the principal organ responsible for the maturation of T cells. Several different subsets of T cells have been discovered, each with a distinct function.

A majority of T cells have a T cell receptor (TCR) existing as a complex of several proteins. The actual T cell receptor is composed of two separate peptide chains, which are produced from the independent T cell receptor alpha and beta (TCRα and TCRβ) genes and are called α- and β-TCR chains. γδ T cells (gamma delta T cells) represent a small subset of T cells that possess a distinct T cell receptor (TCR) on their surface. However, in γδ T cells, the TCR is made up of one γ-chain and one δ-chain. This group of T cells is much less common (2% of total T cells) than the αβ T cells.

All T cells originate from hematopoietic stem cells in the bone marrow. Hematopoietic progenitors derived from hematopoietic stem cells populate the thymus and expand by cell division to generate a large population of immature thymocytes. The earliest thymocytes express neither CD4 nor CD8, and are therefore classed as double-negative (CD4-CD8-) cells. As they progress through their development they become double-positive thymocytes (CD4+CD8+), and finally mature to single-positive (CD4+CD8- or CD4-CD8+) thymocytes that are then released from the thymus to peripheral tissues.

The terms "T cell" and "T lymphocyte" are used interchangeably herein and include T helper cells (CD4+ T cells) and cytotoxic T cells (CTLs, CD8+ T cells) which comprise cytolytic T cells. The term "antigen-specifc T cell" or similar terms relate to a T cell which recognizes the antigen to which the T cell is targeted, in particular when presented on the surface of antigen presenting cells or diseased cells such as cancer cells and preferably exerts effector functions of T cells. T cells are considered to be specific for antigen if the cells kill target cells expressing an antigen. T cell specificity may be evaluated using any of a variety of standard techniques, for example, within a chromium release assay or proliferation assay. Alternatively, synthesis of lymphokines (such as interferon-y) can be measured.

T helper cells assist other white blood cells in immunologic processes, including maturation of B cells into plasma cells and activation of cytotoxic T cells and macrophages, among other functions. These cells are also known as CD4+ T cells because they express the CD4 protein on their surface. Helper T cells become activated when they are presented with peptide antigens by MHC class II molecules that are expressed on the surface of antigen presenting cells (APCs). Once activated, they divide rapidly and secrete small proteins called cytokines that regulate or assist in the active immune response.

Cytotoxic T cells destroy virally infected cells and tumor cells, and are also implicated in transplant rejection. These cells are also known as CD8+ T cells since they express the CD8 glycoprotein at their surface. These cells recognize their targets by binding to antigen associated with MHC class I, which is present on the surface of nearly every cell of the body.

T cell mediated effector functions comprise in the case of a helper T cell (CD4+ T cell) the release of cytokines and/or the activation of CD8⁺ lymphocytes (CTLs) and/or B-cells, and in the case of CTL the elimination of cells, i.e., cells characterized by expression of an antigen, for example, via apoptosis or perforin-mediated cell lysis, production of cytokines such as IFN-γ and TNF-o, and specific cytolytic killing of antigen expressing target cells.

According to the invention, the term "T cell" also includes a cell which can mature into a T cell with suitable stimulation.

T cells may generally be prepared *in vitro* or ex *vivo,* using standard procedures. For example, T cells may be isolated from bone marrow, peripheral blood or a fraction of bone marrow or peripheral blood of a mammal, such as a patient, using a commercially available cell separation system. Alternatively, T cells may be derived from related or unrelated humans, non-human animals, cell lines or cultures. A sample comprising T cells may, for example, be peripheral blood mononuclear cells (PBMC).

The T cells to be used according to the invention may express an endogenous T cell receptor or may lack expression of an endogenous T cell receptor.

### CAR

Nucleic acids such as RNA encoding a CAR may be introduced into T cells or other cells with lytic potential, in particular lymphoid cells.

According to the disclosure, a CAR which when present on a T cell recognizes an antigen such as on the surface of antigen presenting cells or diseased cells such as cancer cells, such that the T cell is stimulated, primed and/or expanded or exerts effector functions as described above.

According to the invention the term "chimeric antigen receptor (CAR)" is synonymous with the terms "chimeric T cell receptor" and "artificial T cell receptor".

Preferably, said CAR is expressed on the surface of the cells.

According to the invention, the term "CAR" (or "chimeric antigen receptor") relates to an artificial receptor comprising a single molecule or a complex of molecules which recognizes, i.e. binds to, a target structure (e.g. an antigen) on a target cell such as a cancer cell (e.g. by binding of an antigen binding domain to an antigen expressed on the surface of the target cell) and may confer specificity onto an immune effector cell such as a T cell expressing said CAR on the cell surface. Such cells do not necessarily require processing and presentation of an antigen for recognition of the target cell but rather may recognize preferably with specificity any antigen present on a target cell. Preferably, recognition of the target structure by a CAR results in activation of an immune effector cell expressing said CAR. A CAR may comprise one or more protein units said protein units comprising one or more domains as described herein. The term "CAR" does not include T cell receptors.

According to the invention, CARs may generally comprise several domains. In one embodiment of all aspects of the invention, the CAR comprises an antigen binding domain, a transmembrane domain, and a T cell signaling domain.

The binding domain recognizes and binds antigen. In one embodiment, a single-chain variable fragment (scFv) derived from a monoclonal antibody is used as a binding domain. Antigen recognition domains which also may be used include among others T cell receptor (TCR) alpha and beta single chains. In fact almost anything that binds a given target with high affinity can be used as an antigen recognition domain. In one embodiment of all aspects of the invention, a CAR comprises an antigen binding domain. In one embodiment, the antigen binding domain is comprised by an exodomain of a CAR. In one embodiment, the antigen binding domain comprises a single-chain variable fragment (scFv) of an antibody to the antigen. In one embodiment, the antigen binding domain comprises a variable region of a heavy chain of an immunoglobulin (VH) with a specificity for the antigen (VH(antigen)) and a variable region of a light chain of an immunoglobulin (VL) with a specificity for the antigen (VL(antigen)). In one embodiment, said heavy chain variable region (VH) and the corresponding light chain variable region (VL) are connected via a peptide linker, preferably a peptide linker comprising the amino acid sequence (GGGGS)3.

In one embodiment of all aspects of the invention, a CAR comprises a transmembrane domain. In one embodiment, the transmembrane domain is a hydrophobic alpha helix that spans the membrane. In one embodiment, the transmembrane domain comprises the CD28 transmembrane domain or a fragment thereof.

The activation signaling domain (or T cell signaling domain) serves to activate cytotoxic lymphocytes upon binding of the CAR to antigen. The identity of the activation signaling domain is limited only in that it has the ability to induce activation of the selected cytotoxic lymphocyte upon binding of the antigen by the CAR. Suitable activation signaling domains include the T cell CD3[zeta] chain and Fc receptor [gamma]. The skilled artisan will understand that sequence variants of these noted activation signaling domains can be used without adversely impacting the invention, where the variants have the same or similar activity as the domain on which they are modeled. Such variants will have at least about 80% sequence identity to the amino acid sequence of the domain from which they are derived.

In one embodiment, the T cell signaling domain is located intracellularly. In one embodiment, the T cell signaling domain comprises CD3-zeta, preferably the endodomain of CD3-zeta, optionally in combination with CD28.

A further domain which may be present is the co-stimulation domain. The co-stimulation domain serves to enhance the proliferation and survival of the cytotoxic lymphocytes upon binding of the CAR to a targeted moiety. The identity of the co-stimulation domain is limited only in that it has the ability to enhance cellular proliferation and survival upon binding of the targeted moiety by the CAR. Suitable co-stimulation domains include CD28, CD137 (4-1BB), a member of the tumor necrosis factor (TNF) receptor family, CD134 (OX40), a member of the TNFR-superfamily of receptors, and CD278 (ICOS), a CD28-superfamily co-stimulatory molecule expressed on activated T cells. The skilled person will understand that sequence variants of these noted co-stimulation domains can be used without adversely impacting the invention, where the variants have the same or similar activity as the domain on which they are modeled. Such variants will have at least about 80% sequence identity to the amino acid sequence of the domain from which they are derived. In some embodiments of the invention, the CAR constructs comprise two co-stimulation domains. While the particular combinations include all possible variations of the four noted domains, specific examples include CD28+CD137 (4-1BB) and CD28+CD134 (0X40).

The CARs of the present invention may comprise the above domains, together in the form of a fusion protein. Such fusion proteins will generally comprise a binding domain, one or more co-stimulation domains, and an activation signaling domain, linked in a N-terminal to C-terminal direction. However, the CARs of the present invention are not limited to this arrangement and other arrangements are acceptable and include a binding domain, an activation signaling domain, and one or more co-stimulation domains. It will be understood that because the binding domain must be free to bind antigen, the placement of the binding domain in the fusion protein will generally be such that display of the region on the exterior of the cell is achieved. In the same manner, because the co-stimulation and activation signaling domains serve to induce activity and proliferation of the cytotoxic lymphocytes, the fusion protein will generally display these two domains in the interior of the cell. The CARs may include additional elements, such as a signal peptide to ensure proper export of the fusion protein to the cells surface, a transmembrane domain to ensure the fusion protein is maintained as an integral membrane protein, and a hinge domain (or spacer region) that imparts flexibility to the binding domain and allows strong binding to antigen.

In one embodiment of all aspects of the invention, a CAR comprises a signal peptide which directs the nascent protein into the endoplasmic reticulum. In one embodiment, the signal peptide precedes the antigen binding domain.

In one embodiment of all aspects of the invention, a CAR comprises a spacer region which links the antigen binding domain to the transmembrane domain. In one embodiment, the spacer region allows the antigen binding domain to orient in different directions to facilitate antigen recognition. In one embodiment, the spacer region comprises the hinge region from IgG1.

In one embodiment of all aspects of the invention, a CAR comprises the structure:
NH2 - signal peptide - antigen binding domain - spacer region - transmembrane domain - T cell signaling domain - COOH.

In one embodiment of all aspects of the invention, a CAR is preferably specific for the antigen to which it is targeted, in particular when present on the surface of a cell such as a diseased cell or an antigen-presenting cell.

In one embodiment of all aspects of the invention, a CAR may be expressed by and/or present on the surface of a T cell, preferably a cytotoxic T cell. In one embodiment, the T cell is reactive with the antigen to which the CAR is targeted.

The cells used in connection with the CAR system of the present invention are preferably T cells, in particular cytotoxic lymphocytes, preferably selected from T cells, in particular cytotoxic T cells, natural killer (NK) cells, and lymphokine-activated killer (LAK) cells. Upon activation, each of these cytotoxic lymphocytes triggers the destruction of target cells. For example, cytotoxic T cells trigger the destruction of target cells by either or both of the following means. First, upon activation, T cells release cytotoxins such as perforin, granzymes, and granulysin. Perforin and granulysin create pores in the target cell, and granzymes enter the cell and trigger a caspase cascade in the cytoplasm that induces apoptosis (programmed cell death) of the cell. Second, apoptosis can be induced via Fas-Fas ligand interaction between the T cells and target cells. The cytotoxic lymphocytes will preferably be autologous cells, although heterologous cells or allogenic cells can be used.

Adoptive cell transfer therapy with T cells expressing chimeric antigen receptors is a promising anticancer therapeutic as CAR-modified T cells can be engineered to target virtually any tumor antigen. For example, patient's T cells may be genetically engineered (genetically modified) to express CARs specifically directed towards antigens on the patient's tumor cells, then infused back into the patient.

According to the invention a CAR may replace the function of a T cell receptor and, in particular, may confer reactivity such as cytolytic activity to a cell such as a T cell. However, in contrast to the binding of the T cell receptor to an antigen peptide-MHC complex, a CAR may bind to an antigen, in particular when expressed on the cell surface.

A variety of methods may be used to introduce CAR constructs into T cells including non-viral-based DNA transfection, transposon-based systems and viral-based systems. Non-viral-based DNA transfection has low risk of insertional mutagenesis. Transposon-based systems can integrate transgenes more efficiently than plasmids that do not contain an integrating element. Viral-based systems include the use of γ-retroviruses and lentiviral vectors. γ-Retroviruses are relatively easy to produce, efficiently and permanently transduce T cells, and have preliminarily proven safe from an integration standpoint in primary human T cells. Lentiviral vectors also efficiently and permanently transduce T cells but are more expensive to manufacture. They are also potentially safer than retrovirus based systems.

In one embodiment of all aspects of the invention, the method further comprises transfecting T cells or T cell progenitors either ex *vivo* or *in vivo* with a nucleic acid encoding the CAR to provide T cells genetically modified to express a CAR.

CAR T cells may be produced *in vivo,* and therefore nearly instantaneously, using nanoparticles targeted to T cells. For example, poly(β-amino ester)-based nanoparticles may be coupled to anti-CD3e f(ab) fragments for binding to CD3 on T cells. For this purpose the anti-CD3e f(ab) fragments may be covalently linked to Polyglutamic acid (PGA). The PGA surrounds the particle core comprising nucleic acids and an excess of poly(β-amino ester) (PBAE) polymer and attaches to it by charge interaction. Upon binding to T cells, these nanoparticles are endocytosed. Their contents, for example plasmid DNA encoding an anti-tumor antigen CAR, may be directed to the T cell nucleus due to the inclusion of peptides containing microtubule-associated sequences (MTAS) and nuclear localization signals (NLSs) covalently linked to the PBAE polymer. The inclusion of transposons flanking the CAR gene expression cassette and a separate plasmid encoding a hyperactive transposase, may allow for the efficient integration of the CAR vector into chromosomes. Such system that allows for the *in vivo* production of CART cells following nanoparticle infusion is described in Smith et al. (2017) Nat. Nanotechnol. 12:813-820.

Another possibility is to use the CRISPRICas9 method to deliberately place a CAR coding sequence at a specific locus. For example, existing T cell receptors (TCR) may be knocked out, while knocking in the CAR and placing it under the dynamic regulatory control of the endogenous promoter that would otherwise moderate TCR expression; c.f., e.g., Eyquem et al. (2017) Nature 543:113-117.

In one embodiment of all aspects of the invention, the T cells genetically modified to express a CAR are stably or transiently transfected with nucleic acid encoding the CAR. Thus, the nucleic acid encoding the CAR is integrated or not integrated into the genome of the T cells.

In one embodiment of all aspects of the invention, the T cells or T cell progenitors are from the subject to be treated. In one embodiment of all aspects of the invention, the T cells or T cell progenitors are from a subject which is different to the subject to be treated.

In one embodiment of all aspects of the invention, the T cells may be autologous, allogeneic or syngeneic to the subject to be treated. The T cells may be genetically modified *in vitro* to express a chimeric antigen receptor (CAR) targeted to the antigen.

In one embodiment of all aspects of the invention, the T cells genetically modified to express a CAR are inactivated for expression of an endogenous T cell receptor and/or endogenous HLA.

The term "autologous" is used to describe anything that is derived from the same subject. For example, "autologous transplant" refers to a transplant of tissue or organs derived from the same subject. Such procedures are advantageous because they overcome the immunological barrier which otherwise results in rejection.

The term "allogeneic" is used to describe anything that is derived from different individuals of the same species. Two or more individuals are said to be allogeneic to one another when the genes at one or more loci are not identical.

The term "syngeneic" is used to describe anything that is derived from individuals or tissues having identical genotypes, i.e., identical twins or animals of the same inbred strain, or their tissues.

The term "heterologous" is used to describe something consisting of multiple different elements. As an example, the transfer of one individual's bone marrow into a different individual constitutes a heterologous transplant. A heterologous gene is a gene derived from a source other than the subject.

### RNA

The term "polynucleotide" or "nucleic acid", as used herein, is intended to include DNA and RNA such as genomic DNA, cDNA, mRNA, recombinantly produced and chemically synthesized molecules. A nucleic acid may be single-stranded or double-stranded. RNA includes *in vitro* transcribed RNA (IVT RNA) or synthetic RNA. According to the invention, a polynucleotide is preferably isolated.

Nucleic acids may be comprised in a vector. The term "vector" as used herein includes any vectors known to the skilled person including plasmid vectors, cosmid vectors, phage vectors such as lambda phage, viral vectors such as adenoviral or baculoviral vectors, or artificial chromosome vectors such as bacterial artificial chromosomes (BAC), yeast artificial chromosomes (YAC), or P1 artificial chromosomes (PAC). Said vectors include expression as well as cloning vectors. Expression vectors comprise plasmids as well as viral vectors and generally contain a desired coding sequence and appropriate DNA sequences necessary for the expression of the operably linked coding sequence in a particular host organism (e.g., bacteria, yeast, plant, insect, or mammal) or in *in vitro* expression systems. Cloning vectors are generally used to engineer and amplify a certain desired DNA fragment and may lack functional sequences needed for expression of the desired DNA fragments.

In one embodiment of all aspects of the invention, nucleic acid encoding a cytokine or encoding an antigen or variant thereof is expressed in cells of the subject treated to provide the cytokine or the antigen or variant thereof. In one embodiment of all aspects of the invention, the nucleic acid is transiently expressed in cells of the mammal. Thus, in one embodiment, the nucleic acid is not integrated into the genome of the cells. In one embodiment of all aspects of the invention, the nucleic acid is RNA, preferably *in vitro* transcribed RNA. In one embodiment of all aspects of the invention, expression of the antigen or variant thereof is at the cell surface.

In one embodiment of all aspects of the invention, the nucleic acid encoding the antigen or variant thereof is expressed in cells of the mammal to provide the antigen or variant thereof for binding by the T cells genetically modified to express a CAR, said binding resulting in stimulation, priming and/or expansion of the T cells genetically modified to express a CAR.

The term "expression" is used according to the invention in its most general meaning and comprises the production of RNA and/or peptides or proteins, e.g. by transcription and/or translation. Expression can be transient or stable. According to the invention, the term expression also includes an "aberrant expression" or "abnormal expression".

According to the invention, the term "nucleic acid encodes" means that the nucleic acid, if present in the appropriate environment, such as within cells can be expressed to produce a protein or peptide it encodes.

The nucleic acids described herein may be recombinant and/or isolated molecules.

An "isolated molecule" as used herein, is intended to refer to a molecule which is substantially free of other molecules such as other cellular material.

The term "recombinant" in the context of the present invention means "made through genetic engineering". Preferably, a "recombinant object" such as a recombinant cell in the context of the present invention is not occurring naturally.

The term "naturally occurring" as used herein refers to the fact that an object can be found in nature. For example, a peptide or nucleic acid that is present in an organism (including viruses) and can be isolated from a source in nature and which has not been intentionally modified by man in the laboratory is naturally occurring.

The term "transfection" relates to the introduction of nucleic acids, in particular RNA, into a cell. For purposes of the present invention, the term "transfection" also includes the introduction of a nucleic acid into a cell or the uptake of a nucleic acid by such cell, wherein the cell may be present in a subject, e.g., a patient. Thus, according to the present invention, a cell for transfection of a nucleic acid described herein can be present *in vitro* or *in vivo,* e.g. the cell can form part of an organ, a tissue and/or an organism of a patient. According to the invention, transfection can be transient or stable. For some applications of transfection, it is sufficient if the transfected genetic material is only transiently expressed. Since the nucleic acid introduced in the transfection process is usually not integrated into the nuclear genome, the foreign nucleic acid will be diluted through mitosis or degraded. Cells allowing episomal amplification of nucleic acids greatly reduce the rate of dilution. If it is desired that the transfected nucleic acid actually remains in the genome of the cell and its daughter cells, a stable transfection must occur. RNA can be transfected into cells to transiently express its coded protein.

In one embodiment of all aspects of the invention, the nucleic acid encoding a cytokine or encoding an antigen or variant thereof is formulated in a delivery vehicle such as in particles. In one embodiment, the delivery vehicle comprises at least one lipid. In one embodiment, the at least one lipid comprises at least one cationic lipid. In one embodiment, the lipid forms a complex with and/or encapsulates the nucleic acid. In one embodiment, the lipid is comprised in a vesicle encapsulating the nucleic acid. In one embodiment of all aspects of the invention, the nucleic acid is formulated in liposomes.

In the present disclosure, the term "RNA" relates to a nucleic acid molecule which includes ribonucleotide residues. In preferred embodiments, the RNA contains all or a majority of ribonucleotide residues. As used herein, "ribonucleotide" refers to a nucleotide with a hydroxyl group at the 2'-position of a β-D-ribofuranosyl group. RNA encompasses without limitation, double stranded RNA, single stranded RNA, isolated RNA such as partially purified RNA, essentially pure RNA, synthetic RNA, recombinantly produced RNA, as well as modified RNA that differs from naturally occurring RNA by the addition, deletion, substitution and/or alteration of one or more nucleotides. Such alterations may refer to addition of non-nucleotide material to internal RNA nucleotides or to the end(s) of RNA. It is also contemplated herein that nucleotides in RNA may be non-standard nucleotides, such as chemically synthesized nucleotides or deoxynucleotides. For the present disclosure, these altered RNAs are considered analogs of naturally-occurring RNA.

In certain embodiments of the present disclosure, the RNA is messenger RNA (mRNA) that relates to a RNA transcript which encodes a peptide or protein. As established in the art, mRNA generally contains a 5' untranslated region (5'-UTR), a peptide coding region and a 3' untranslated region (3'-UTR). In some embodiments, the RNA is produced by *in vitro* transcription or chemical synthesis. In one embodiment, the mRNA is produced by *in vitro* transcription using a DNA template where DNA refers to a nucleic acid that contains deoxyribonucleotides.

In one embodiment, RNA is *in vitro* transcribed RNA (IVT-RNA) and may be obtained by *in vitro* transcription of an appropriate DNA template. The promoter for controlling transcription can be any promoter for any RNA polymerase. A DNA template for *in vitro* transcription may be obtained by cloning of a nucleic acid, in particular cDNA, and introducing it into an appropriate vector for *in vitro* transcription. The cDNA may be obtained by reverse transcription of RNA.

In one embodiment, the RNA may have modified ribonucleotides. Examples of modified ribonucleotides include, without limitation, 5-methylcytidine, pseudouridine and/or 1-methyl-pseudouridine.

In some embodiments, the RNA according to the present disclosure comprises a 5'-cap. In one embodiment, the RNA of the present disclosure does not have uncapped 5'-triphosphates. In one embodiment, the RNA may be modified by a 5'- cap analog. The term "5'-cap" refers to a structure found on the 5'-end of an mRNA molecule and generally consists of a guanosine nucleotide connected to the mRNA via a 5' to 5' triphosphate linkage. In one embodiment, this guanosine is methylated at the 7-position. Providing an RNA with a 5'-cap or 5'-cap analog may be achieved by *in vitro* transcription, in which the 5'-cap is co-transcriptionally expressed into the RNA strand, or may be attached to RNA post-transcriptionally using capping enzymes.

In some embodiments, RNA according to the present disclosure comprises a 5'-UTR and/or a 3'-UTR. The term "untranslated region" or "UTR" relates to a region in a DNA molecule which is transcribed but is not translated into an amino acid sequence, or to the corresponding region in an RNA molecule, such as an mRNA molecule. An untranslated region (UTR) can be present 5' (upstream) of an open reading frame (5'-UTR) and/or 3' (downstream) of an open reading frame (3'-UTR). A 5'-UTR, if present, is located at the 5' end, upstream of the start codon of a protein-encoding region. A 5'-UTR is downstream of the 5'-cap (if present), e.g. directly adjacent to the 5'-cap. A 3'-UTR, if present, is located at the 3' end, downstream of the termination codon of a protein-encoding region, but the term "3'-UTR" does preferably not include the poly(A) tail. Thus, the 3'-UTR is upstream of the poly(A) sequence (if present), e.g. directly adjacent to the poly(A) sequence.

In some embodiments, the RNA according to the present disclosure comprises a 3'-poly(A) sequence. The term "poly(A) sequence" relates to a sequence of adenyl (A) residues which typically is located at the 3'-end of a RNA molecule. According to the disclosure, in one embodiment, a poly(A) sequence comprises at least about 20, at least about 40, at least about 80, or at least about 100, and up to about 500, up to about 400, up to about 300, up to about 200, or up to about 150 A nucleotides, and in particular about 120 A nucleotides.

In the context of the present disclosure, the term "transcription" relates to a process, wherein the genetic code in a DNA sequence is transcribed into RNA. Subsequently, the RNA may be translated into peptide or protein.

With respect to RNA, the term "expression" or "translation" relates to the process in the ribosomes of a cell by which a strand of mRNA directs the assembly of a sequence of amino acids to make a peptide or protein.

According to the disclosure, the term "RNA encodes" means that the RNA, if present in the appropriate environment, such as within cells of a target tissue, can direct the assembly of amino acids to produce the peptide or protein it encodes during the process of translation. In one embodiment, RNA is able to interact with the cellular translation machinery allowing translation of the peptide or protein. A cell may produce the encoded peptide or protein intracellularly (e.g. in the cytoplasm and/or in the nucleus), may secrete the encoded peptide or protein, or may produce it on the surface.

As used herein, the terms "linked," "fused", or "fusion" are used interchangeably. These terms refer to the joining together of two or more elements or components or domains.

As used herein, "half-life" refers to the time taken for the serum or plasma concentration of a peptide or protein to reduce by 50%, *in vivo,* for example due to degradation and/or clearance or sequestration by natural mechanisms. An extended-PK cytokine such as extended-PK interleukin (IL) suitable for use herein is stabilized *in vivo* and its half-life increased by, e.g., fusion to serum albumin (e.g., HSA or MSA), which resist degradation and/or clearance or sequestration. The half-life can be determined in any manner known per se, such as by pharmacokinetic analysis. Suitable techniques will be clear to the person skilled in the art, and may for example generally involve the steps of suitably administering a suitable dose of the amino acid sequence or compound to a subject; collecting blood samples or other samples from said subject at regular intervals; determining the level or concentration of the amino acid sequence or compound in said blood sample; and calculating, from (a plot of) the data thus obtained, the time until the level or concentration of the amino acid sequence or compound has been reduced by 50% compared to the initial level upon dosing. Further details are provided in, e.g., standard handbooks, such as Kenneth, A. et al., Chemical Stability of Pharmaceuticals: A Handbook for Pharmacists and in Peters et al., Pharmacokinetic Analysis: A Practical Approach (1996). Reference is also made to Gibaldi, M. et al., Pharmacokinetics, 2nd Rev. Edition, Marcel Dekker (1982).

### Cytokines

Cytokines are a category of small proteins (~5-20 kDa) that are important in cell signalling. Their release has an effect on the behavior of cells around them. Cytokines are involved in autocrine signalling, paracrine signalling and endocrine signalling as immunomodulating agents. Cytokines include chemokines, interferons, interleukins, lymphokines, and tumour necrosis factors but generally not hormones or growth factors (despite some overlap in the terminology). Cytokines are produced by a broad range of cells, including immune cells like macrophages, B lymphocytes, T lymphocytes and mast cells, as well as endothelial cells, fibroblasts, and various stromal cells. A given cytokine may be produced by more than one type of cell. Cytokines act through receptors, and are especially important in the immune system; cytokines modulate the balance between humoral and cell-based immune responses, and they regulate the maturation, growth, and responsiveness of particular cell populations. Some cytokines enhance or inhibit the action of other cytokines in complex ways.

### IL2

Interleukin-2 (IL2) is a cytokine that induces proliferation of antigen-activated T cells and stimulates natural killer (NK) cells. The biological activity of IL2 is mediated through a multi-subunit IL2 receptor complex (IL2R) of three polypeptide subunits that span the cell membrane: p55 (IL2Ra, the alpha subunit, also known as CD25 in humans), p75 (IL2Rβ, the beta subunit, also known as CD122 in humans) and p64 (IL2Ry, the gamma subunit, also known as CD 132 in humans). T cell response to IL2 depends on a variety of factors, including: (1) the concentration of IL2; (2) the number of IL2R molecules on the cell surface; and (3) the number of IL2R occupied by IL2 (i.e., the affinity of the binding interaction between IL2 and IL2R (Smith, "Cell Growth Signal Transduction is Quantal" In Receptor Activation by Antigens, Cytokines, Hormones, and Growth Factors 766:263-271, 1995)). The IL2:IL2R complex is internalized upon ligand binding and the different components undergo differential sorting. When administered as an intravenous (i.v.) bolus, IL2 has a rapid systemic clearance (an initial clearance phase with a half-life of 12.9 minutes followed by a slower clearance phase with a half-life of 85 minutes) (Konrad et al., Cancer Res. 50:2009-2017, 1990).

Outcomes of systemic IL2 administration in cancer patients are far from ideal. While 15 to 20 percent of patients respond objectively to high-dose IL2, the great majority do not, and many suffer severe, life-threatening side effects, including nausea, confusion, hypotension, and septic shock. The severe toxicity associated with high-dose IL2 treatment is largely attributable to the activity of natural killer (NK) cells. Attempts to reduce serum concentration by reducing dose and adjusting dosing regimen have been attempted, and while less toxic, such treatments were also less efficacious.

According to the disclosure, in certain embodiments, IL2 is attached to a pharmacokinetic modifying group. The resulting molecule, hereafter referred to as "extended-pharmacokinetic (PK) IL2," has a prolonged circulation half-life relative to free IL2. The prolonged circulation half-life of extended-PK IL2 permits *in vivo* serum IL2 concentrations to be maintained within a therapeutic range, potentially leading to the enhanced activation of many types of immune cells, including T cells. Because of its favorable pharmacokinetic profile, extended-PK IL2 can be dosed less frequently and for longer periods of time when compared with unmodified IL2.

According to the disclosure, IL2 (optionally as a portion of extended-PK IL2) may be naturally occurring IL2 or a fragment or variant thereof. IL2 may be human IL2 and may be derived from any vertebrate, especially any mammal. In one embodiment, IL2 comprises the amino acid sequence of SEQ ID NO: 1 or an amino acid sequence that is at least 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 1. In one embodiment, IL2 or a IL2 fragment or variant binds to the IL2 receptor, or a subunit of the IL2 receptor such as the alpha subunit and/or the beta/gamma subunit.

In certain embodiments, the IL2 moiety of the extended-PK IL2 is human IL2. In other embodiments, the IL2 moiety of the extended-PK IL2 is a fragment or variant of human IL2.

In certain embodiments described herein, IL2 is fused to a heterologous polypeptide (i.e., a polypeptide that is not IL2). The heterologous polypeptide can increase the circulating half-life of IL2. As discussed in further detail infra, the polypeptide that increases the circulating half-life may be serum albumin, such as human (e.g., SEQ ID NO: 4) or mouse (e.g., SEQ ID NO: 8, 11) serum albumin.

### IL7

IL7 is a hematopoietic growth factor secreted by stromal cells in the bone marrow and thymus. It is also produced by keratinocytes, dendritic cells, hepatocytes, neurons, and epithelial cells, but is not produced by normal lymphocytes. IL7 is a cytokine important for B and T cell development. IL7 cytokine and the hepatocyte growth factor form a heterodimer that functions as a pre-pro-B cell growth-stimulating factor. Knockout studies in mice suggested that IL7 plays an essential role in lymphoid cell survival.

IL7 binds to the IL7 receptor, a heterodimer consisting of IL7 receptor α and common γ chain receptor. Binding results in a cascade of signals important for T cell development within the thymus and survival within the periphery. Knockout mice which genetically lack IL7 receptor exhibit thymic atrophy, arrest of T cell development at the double positive stage, and severe lymphopenia. Administration of IL7 to mice results in an increase in recent thymic emigrants, increases in B and T cells, and increased recovery of T cells after cyclophosphamide administration or after bone marrow transplantation.

According to the disclosure, in certain embodiments, IL7 is attached to a pharmacokinetic modifying group. The resulting molecule, hereafter referred to as "extended-pharmacokinetic (PK) IL7," has a prolonged circulation half-life relative to free IL7. The prolonged circulation half-life of extended-PK IL7 permits *in vivo* serum IL7 concentrations to be maintained within a therapeutic range, potentially leading to the enhanced survival of many types of immune cells, including T cells. Because of its favorable pharmacokinetic profile, extended-PK IL7 can be dosed less frequently and for longer periods of time when compared with unmodified IL7.

According to the disclosure, IL7 (optionally as a portion of extended-PK IL7) may be naturally occurring IL7 or a fragment or variant thereof. IL7 may be human IL7 and may be derived from any vertebrate, especially any mammal. In one embodiment, IL7 comprises the amino acid sequence of SEQ ID NO: 2 or an amino acid sequence that is at least 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 2. In one embodiment, IL7 or a IL7 fragment or variant binds to the IL7 receptor.

In certain embodiments, the IL7 moiety of the extended-PK IL7 is human IL7. In other embodiments, the IL7 moiety of the extended-PK IL7 is a fragment or variant of human IL7.

In certain embodiments described herein, IL7 is fused to a heterologous polypeptide (i.e., a polypeptide that is not IL7). The heterologous polypeptide can increase the circulating half-life of IL7. As discussed in further detail infra, the polypeptide that increases the circulating half-life may be serum albumin, such as human (e.g., SEQ ID NO: 4) or mouse (e.g., SEQ ID NO: 8, 11) serum albumin.

### IL21

Interleukin-21 (IL21) is a cytokine that has potent regulatory effects on cells of the immune system, including natural killer (NK) cells and cytotoxic T cells. This cytokine induces cell division/proliferation in its target cells. IL21 is expressed in activated human CD4+ T cells but not in most other tissues. In addition, IL21 expression is up-regulated in Th2 and Th17 subsets of T helper cells, as well as T follicular cells. Furthermore, IL21 is expressed in NK T cells regulating the function of these cells. Interleukin21 is also produced by Hodgkin's lymphoma (HL) cancer cells.

The IL21 receptor (IL21R) is expressed on the surface of T, B and NK cells. IL21R is similar in structure to the receptors for other type I cytokines like IL2 or IL-15 and requires dimerization with the common gamma chain (γc) in order to bind IL-21. When bound to IL21, the IL21 receptor acts through the Jak/STAT pathway, utilizing Jak1 and Jak3 and a STAT3 homodimer to activate its target genes.

According to the disclosure, in certain embodiments, IL21 is attached to a pharmacokinetic modifying group. The resulting molecule, hereafter referred to as "extended-pharmacokinetic (PK) IL21," has a prolonged circulation half-life relative to free IL21. The prolonged circulation half-life of extended-PK IL21 permits *in vivo* serum IL21 concentrations to be maintained within a therapeutic range, potentially leading to the enhanced activation of many types of immune cells, including T cells. Because of its favorable pharmacokinetic profile, extended-PK IL21 can be dosed less frequently and for longer periods of time when compared with unmodified IL21.

According to the disclosure, IL21 (optionally as a portion of extended-PK IL21) may be naturally occurring IL21 or a fragment or variant thereof. IL21 may be human IL21 and may be derived from any vertebrate, especially any mammal. In one embodiment, IL21 comprises the amino acid sequence of SEQ ID NO: 3 or an amino acid sequence that is at least 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 3. In one embodiment, IL21 or a IL21 fragment or variant binds to the IL21 receptor.

In certain embodiments, the IL21 moiety of the extended-PK IL21 is human IL21. In other embodiments, the IL21 moiety of the extended-PK IL21 is a fragment or variant of human IL21.

In certain embodiments described herein, IL21 is fused to a heterologous polypeptide (i.e., a polypeptide that is not IL21). The heterologous polypeptide can increase the circulating half-life of IL21. As discussed in further detail infra, the polypeptide that increases the circulating half-life may be serum albumin, such as human (e.g., SEQ ID NO: 4) or mouse (e.g., SEQ ID NO: 8, 11) serum albumin.

### Extended-PK group

The cytokines, e.g. interleukins, described herein, such as IL2, IL7 or IL21, may be fused to an extended-PK group, which increases circulation half-life. Non-limiting examples of extended-PK groups are described infra. It should be understood that other PK groups that increase the circulation half-life of cytokines, or variants thereof, are also applicable to the present disclosure. In certain embodiments, the extended-PK group is a serum albumin domain (e.g., mouse serum albumin, human serum albumin).

As used herein, the term "PK" is an acronym for "pharmacokinetic" and encompasses properties of a compound including, by way of example, absorption, distribution, metabolism, and elimination by a subject. As used herein, an "extended-PK group" refers to a protein, peptide, or moiety that increases the circulation half-life of a biologically active molecule when fused to or administered together with the biologically active molecule. Examples of an extended-PK group include serum albumin (e.g., HSA), Fc or Fc fragments and variants thereof, transferrin and variants thereof, and human serum albumin (HSA) binders (as disclosed in U.S. Publication Nos. 2005/0287153 and 2007/0003549). Other exemplary extended-PK groups are disclosed in Kontermann et al., Current Opinion in Biotechnology 2011; 22: 868-876, which is herein incorporated by reference in its entirety. As used herein, an "extended-PK cytokine" refers to a cytokine moiety in combination with an extended-PK group. In one embodiment, the extended-PK cytokine is a fusion protein in which a cytokine moiety is linked or fused to an extended-PK group. As used herein, an "extended-PK IL" refers to an interleukin (IL) moiety in combination with an extended-PK group. In one embodiment, the extended-PK IL is a fusion protein in which an IL moiety is linked or fused to an extended-PK group. An exemplary fusion protein is an HSA/IL2 fusion in which an IL2 moiety is fused with HSA. Another exemplary fusion protein is an HSA/IL7 fusion in which an IL7 moiety is fused with HSA. Another exemplary fusion protein is an HSA/IL21 fusion in which an IL21 moiety is fused with HSA.

In certain embodiments, the serum half-life of an extended-PK cytokine is increased relative to the cytokine alone (i.e., the cytokine not fused to an extended-PK group). In certain embodiments, the serum half-life of the extended-PK cytokine is at least 20, 40, 60, 80, 100, 120, 150, 180, 200, 400, 600, 800, or 1000% longer relative to the serum half-life of the cytokine alone. In certain embodiments, the serum half-life of the extended-PK cytokine is at least 1.5-fold, 2-fold, 2.5-fold, 3-fold, 3.5 fold, 4-fold, 4.5-fold, 5-fold, 6-fold, 7-fold, 8-fold, 10- fold, 12-fold, 13-fold, 15-fold, 17-fold, 20-fold, 22- fold, 25-fold, 27-fold, 30-fold, 35-fold, 40-fold, or 50-fold greater than the serum half-life of the cytokine alone. In certain embodiments, the serum half-life of the extended-PK cytokine is at least 10 hours, 15 hours, 20 hours, 25 hours, 30 hours, 35 hours, 40 hours, 50 hours, 60 hours, 70 hours, 80 hours, 90 hours, 100 hours, 110 hours, 120 hours, 130 hours, 135 hours, 140 hours, 150 hours, 160 hours, or 200 hours.

In certain embodiments, the extended-PK group includes serum albumin, or fragments thereof or variants of the serum albumin or fragments thereof (all of which for the purpose of the present disclosure are comprised by the term "albumin"). Polypeptides described herein may be fused to albumin (or a fragment or variant thereof) to form albumin fusion proteins. Such albumin fusion proteins are described in U.S. Publication No. 20070048282.

As used herein, "albumin fusion protein" refers to a protein formed by the fusion of at least one molecule of albumin (or a fragment or variant thereof) to at least one molecule of a protein such as a therapeutic protein, in particular IL2, IL7 or IL21 (or fragment or variant thereof). The albumin fusion protein may be generated by translation of a nucleic acid in which a polynucleotide encoding a therapeutic protein is joined in-frame with a polynucleotide encoding an albumin. The therapeutic protein and albumin, once part of the albumin fusion protein, may each be referred to as a "portion", "region" or "moiety" of the albumin fusion protein (e.g., a "therapeutic protein portion" or an "albumin protein portion"). In a highly preferred embodiment, an albumin fusion protein comprises at least one molecule of a therapeutic protein (including, but not limited to a mature form of the therapeutic protein) and at least one molecule of albumin (including but not limited to a mature form of albumin). In one embodiment, an albumin fusion protein is processed by a host cell such as a cell of the target organ for administered RNA, e.g. a liver cell, and secreted into the circulation. Processing of the nascent albumin fusion protein that occurs in the secretory pathways of the host cell used for expression of the RNA may include, but is not limited to signal peptide cleavage; formation of disulfide bonds; proper folding; addition and processing of carbohydrates (such as for example, N- and O-linked glycosylation); specific proteolytic cleavages; and/or assembly into multimeric proteins. An albumin fusion protein is preferably encoded by RNA in a non-processed form which in particular has a signal peptide at its N-terminus and following secretion by a cell is preferably present in the processed form wherein in particular the signal peptide has been cleaved off. In a most preferred embodiment, the "processed form of an albumin fusion protein" refers to an albumin fusion protein product which has undergone N-terminal signal peptide cleavage, herein also referred to as a "mature albumin fusion protein".

In preferred embodiments, albumin fusion proteins comprising a therapeutic protein have a higher plasma stability compared to the plasma stability of the same therapeutic protein when not fused to albumin. Plasma stability typically refers to the time period between when the therapeutic protein is administered *in vivo* and carried into the bloodstream and when the therapeutic protein is degraded and cleared from the bloodstream, into an organ, such as the kidney or liver, that ultimately clears the therapeutic protein from the body. Plasma stability is calculated in terms of the half-life of the therapeutic protein in the bloodstream. The half-life of the therapeutic protein in the bloodstream can be readily determined by common assays known in the art.

As used herein, "albumin" refers collectively to albumin protein or amino acid sequence, or an albumin fragment or variant, having one or more functional activities (e.g., biological activities) of albumin. In particular, "albumin" refers to human albumin or fragments or variants thereof especially the mature form of human albumin, or albumin from other vertebrates or fragments thereof, or variants of these molecules. The albumin may be derived from any vertebrate, especially any mammal, for example human, cow, sheep, or pig. Non-mammalian albumins include, but are not limited to, hen and salmon. The albumin portion of the albumin fusion protein may be from a different animal than the therapeutic protein portion.

In certain embodiments, the albumin is human serum albumin (HSA), or fragments or variants thereof, such as those disclosed in US 5,876,969, WO 2011/124718, WO 2013/075066, and WO 2011/0514789.

The terms, human serum albumin (HSA) and human albumin (HA) are used interchangeably herein. The terms, "albumin and "serum albumin" are broader, and encompass human serum albumin (and fragments and variants thereof) as well as albumin from other species (and fragments and variants thereof).

As used herein, a fragment of albumin sufficient to prolong the therapeutic activity or plasma stability of the therapeutic protein refers to a fragment of albumin sufficient in length or structure to stabilize or prolong the therapeutic activity or plasma stability of the protein so that the plasma stability of the therapeutic protein portion of the albumin fusion protein is prolonged or extended compared to the plasma stability in the non-fusion state.

The albumin portion of the albumin fusion proteins may comprise the full length of the albumin sequence, or may include one or more fragments thereof that are capable of stabilizing or prolonging the therapeutic activity or plasma stability. Such fragments may be of 10 or more amino acids in length or may include about 15, 20, 25, 30, 50, or more contiguous amino acids from the albumin sequence or may include part or all of specific domains of albumin. For instance, one or more fragments of HSA spanning the first two immunoglobulin-like domains may be used. In a preferred embodiment, the HSA fragment is the mature form of HSA.

Generally speaking, an albumin fragment or variant will be at least 100 amino acids long, preferably at least 150 amino acids long.

According to the disclosure, albumin may be naturally occurring albumin or a fragment or variant thereof. Albumin may be human albumin and may be derived from any vertebrate, especially any mammal. In one embodiment, albumin comprises the amino acid sequence of SEQ ID NO: 4 or an amino acid sequence that is at least 80%, 81%, 82%, 83%, 84%, 85%, 86%, 87%, 88%, 89%, 90%, 91%, 92%, 93%, 94%, 95%, 96%, 97%, 98%, or 99% identical to SEQ ID NO: 4.

Preferably, the albumin fusion protein comprises albumin as the N-terminal portion, and a therapeutic protein as the C-terminal portion. Alternatively, an albumin fusion protein comprising albumin as the C-terminal portion, and a therapeutic protein as the N-terminal portion may also be used. In other embodiments, the albumin fusion protein has a therapeutic protein fused to both the N-terminus and the C-terminus of albumin. In a preferred embodiment, the therapeutic proteins fused at the N- and C-termini are the same therapeutic proteins. In another preferred embodiment, the therapeutic proteins fused at the N- and C-termini are different therapeutic proteins. In one embodiment, the different therapeutic proteins may be useful to treat or prevent the same or a related disease, disorder, or condition. In one embodiment, the different therapeutic proteins are both cytokines.

In one embodiment, the therapeutic protein(s) is (are) joined to the albumin through (a) peptide linker(s). A linker peptide between the fused portions may provide greater physical separation between the moieties and thus maximize the accessibility of the therapeutic protein portion, for instance, for binding to its cognate receptor. The linker peptide may consist of amino acids such that it is flexible or more rigid. The linker sequence may be cleavable by a protease or chemically.

As used herein, the term "Fc region" refers to the portion of a native immunoglobulin formed by the respective Fc domains (or Fc moieties) of its two heavy chains. As used herein, the term "Fc domain" refers to a portion or fragment of a single immunoglobulin (Ig) heavy chain wherein the Fc domain does not comprise an Fv domain. In certain embodiments, an Fc domain begins in the hinge region just upstream of the papain cleavage site and ends at the C-terminus of the antibody. Accordingly, a complete Fc domain comprises at least a hinge domain, a CH2 domain, and a CH3 domain. In certain embodiments, an Fc domain comprises at least one of: a hinge (e.g., upper, middle, and/or lower hinge region) domain, a CH2 domain, a CH3 domain, a CH4 domain, or a variant, portion, or fragment thereof. In certain embodiments, an Fc domain comprises a complete Fc domain (i.e., a hinge domain, a CH2 domain, and a CH3 domain). In certain embodiments, an Fc domain comprises a hinge domain (or portion thereof) fused to a CH3 domain (or portion thereof). In certain embodiments, an Fc domain comprises a CH2 domain (or portion thereof) fused to a CH3 domain (or portion thereof). In certain embodiments, an Fc domain consists of a CH3 domain or portion thereof. In certain embodiments, an Fc domain consists of a hinge domain (or portion thereof) and a CH3 domain (or portion thereof). In certain embodiments, an Fc domain consists of a CH2 domain (or portion thereof) and a CH3 domain. In certain embodiments, an Fc domain consists of a hinge domain (or portion thereof) and a CH2 domain (or portion thereof). In certain embodiments, an Fc domain lacks at least a portion of a CH2 domain (e.g., all or part of a CH2 domain). An Fc domain herein generally refers to a polypeptide comprising all or part of the Fc domain of an immunoglobulin heavy-chain. This includes, but is not limited to, polypeptides comprising the entire CH1, hinge, CH2, and/or CH3 domains as well as fragments of such peptides comprising only, e.g., the hinge, CH2, and CH3 domain. The Fc domain may be derived from an immunoglobulin of any species and/or any subtype, including, but not limited to, a human IgG1, IgG2, IgG3, IgG4, IgD, IgA, IgE, or IgM antibody. The Fc domain encompasses native Fc and Fc variant molecules. As set forth herein, it will be understood by one of ordinary skill in the art that any Fc domain may be modified such that it varies in amino acid sequence from the native Fc domain of a naturally occurring immunoglobulin molecule. In certain embodiments, the Fc domain has reduced effector function (e.g., FcγR binding).

The Fc domains of a polypeptide described herein may be derived from different immunoglobulin molecules. For example, an Fc domain of a polypeptide may comprise a CH2 and/or CH3 domain derived from an IgG1 molecule and a hinge region derived from an IgG3 molecule. In another example, an Fc domain can comprise a chimeric hinge region derived, in part, from an IgG1 molecule and, in part, from an IgG3 molecule. In another example, an Fc domain can comprise a chimeric hinge derived, in part, from an IgG1 molecule and, in part, from an IgG4 molecule.

In certain embodiments, an extended-PK group includes an Fc domain or fragments thereof or variants of the Fc domain or fragments thereof (all of which for the purpose of the present disclosure are comprised by the term "Fc domain"). The Fc domain does not contain a variable region that binds to antigen. Fc domains suitable for use in the present disclosure may be obtained from a number of different sources. In certain embodiments, an Fc domain is derived from a human immunoglobulin. In certain embodiments, the Fc domain is from a human IgG1 constant region. It is understood, however, that the Fc domain may be derived from an immunoglobulin of another mammalian species, including for example, a rodent (e.g. a mouse, rat, rabbit, guinea pig) or non- human primate (e.g. chimpanzee, macaque) species.

Moreover, the Fc domain (or a fragment or variant thereof) may be derived from any immunoglobulin class, including IgM, IgG, IgD, IgA, and IgE, and any immunoglobulin isotype, including IgG1, IgG2, IgG3, and IgG4.

A variety of Fc domain gene sequences (e.g., mouse and human constant region gene sequences) are available in the form of publicly accessible deposits. Constant region domains comprising an Fc domain sequence can be selected lacking a particular effector function and/or with a particular modification to reduce immunogenicity. Many sequences of antibodies and antibody-encoding genes have been published and suitable Fc domain sequences (e.g. hinge, CH2, and/or CH3 sequences, or fragments or variants thereof) can be derived from these sequences using art recognized techniques.

In certain embodiments, the extended-PK group is a serum albumin binding protein such as those described in US2005/0287153, US2007/0003549, US2007/0178082, US2007/0269422, US201010113339, WO2009/083804, and WO2009/133208, which are herein incorporated by reference in their entirety. In certain embodiments, the extended-PK group is transferrin, as disclosed in US 7,176,278 and US 8,158,579, which are herein incorporated by reference in their entirety. In certain embodiments, the extended-PK group is a serum immunoglobulin binding protein such as those disclosed in US2007/0178082, which is herein incorporated by reference in its entirety. In certain embodiments, the extended-PK group is a fibronectin (Fn)-based scaffold domain protein that binds to serum albumin, such as those disclosed in US2012/0094909, which is herein incorporated by reference in its entirety. Methods of making fibronectin-based scaffold domain proteins are also disclosed in US2012/0094909. A non-limiting example of a Fn3-based extended-PK group is Fn3(HSA), i.e., a Fn3 protein that binds to human serum albumin.

In certain aspects, the extended-PK cytokine such as extended-PK IL, suitable for use according to the disclosure, can employ one or more peptide linkers. As used herein, the term "peptide linker" refers to a peptide or polypeptide sequence which connects two or more domains (e.g., the extended-PK moiety and an IL moiety such as IL2, IL7 or IL21) in a linear amino acid sequence of a polypeptide chain. For example, peptide linkers may be used to connect an IL2 moiety to a HSA domain. In another embodiment, peptide linkers may be used to connect an IL7 moiety to a HSA domain. In another embodiment, peptide linkers may be used to connect an IL21 moiety to a HSA domain.

Linkers suitable for fusing the extended-PK group to e.g. IL2, IL7 or IL21 are well known in the art. Exemplary linkers include glycine-serine-polypeptide linkers, glycine-proline-polypeptide linkers, and proline-alanine polypeptide linkers. In certain embodiments, the linker is a glycine-serine-polypeptide linker, i.e., a peptide that consists of glycine and serine residues.

### Antigen

The peptide and protein antigens suitable for use according to the disclosure, i.e., the antigen or variant thereof, typically include a peptide or protein comprising an epitope for inducing an immune response. The peptide or protein or epitope may be derived from a target antigen, i.e. the antigen against which an immune response is to be elicited. For example, the peptide or protein antigen or the epitope contained within the peptide or protein antigen may be a target antigen or a fragment or variant of a target antigen.

A peptide and protein antigen which is administered or which is encoded by the nucleic acid, in particular RNA which is administered, i.e., a vaccine antigen, preferably results in stimulation, priming and/or expansion of T cells genetically modified to express a CAR in the subject being administered the peptide or protein antigen or nucleic acid. Said stimulated, primed and/or expanded T cells are preferably directed against a target antigen, in particular a target antigen expressed by diseased cells, tissues and/or organs, i.e., a disease-associated antigen. Thus, a vaccine antigen may comprise the disease-associated antigen, or a fragment or variant thereof. In one embodiment, such fragment or variant is immunologically equivalent to the disease-associated antigen. In the context of the present disclosure, the term "fragment of an antigen" or "variant of an antigen" means an agent which results in stimulation, priming and/or expansion of CAR-engineered T cells which stimulated, primed and/or expanded T cells target the antigen, i.e. a disease-associated antigen, in particular when presented by diseased cells, tissues and/or organs. Thus, the vaccine antigen may correspond to or may comprise the disease-associated antigen, may correspond to or may comprise a fragment of the disease-associated antigen or may correspond to or may comprise an antigen which is homologous to the disease-associated antigen or a fragment thereof. If the vaccine antigen comprises a fragment of the disease-associated antigen or an amino acid sequence which is homologous to a fragment of the disease-associated antigen said fragment or amino acid sequence may comprise an epitope of the disease-associated antigen to which the CAR of the CAR-engineered T cells is targeted or a sequence which is homologous to an epitope of the disease-associated antigen. Thus, according to the disclosure, a vaccine antigen may comprise an immunogenic fragment of a disease-associated antigen or an amino acid sequence being homologous to an immunogenic fragment of a disease-associated antigen. An "immunogenic fragment of an antigen" according to the disclosure preferably relates to a fragment of an antigen which is capable of stimulating, priming and/or expanding T cells carrying a CAR binding to the antigen or cells expressing the antigen. It is preferred that the vaccine antigen (similar to the disease-associated antigen) can be expressed on the surface of a cell such as an antigen-presenting cell so as to provide the relevant epitope for binding by CAR-engineered T cells. The vaccine antigen may be a recombinant antigen.

The term "immunologically equivalent" means that the immunologically equivalent molecule such as the immunologically equivalent amino acid sequence exhibits the same or essentially the same immunological properties and/or exerts the same or essentially the same immunological effects, e.g., with respect to the type of the immunological effect. In the context of the present disclosure, the term "immunologically equivalent" is preferably used with respect to the immunological effects or properties of antigens or antigen variants used for immunization. For example, an amino acid sequence is immunologically equivalent to a reference amino acid sequence if said amino acid sequence when exposed to the immune system of a subject such as T cells binding to the reference amino acid sequence or cells expressing the reference amino acid sequence induces an immune reaction having a specificity of reacting with the reference amino acid sequence. Thus, a molecule which is immunologically equivalent to an antigen exhibits the same or essentially the same properties and/or exerts the same or essentially the same effects regarding the stimulation, priming and/or expansion of T cells as the antigen to which the T cells are targeted.

The term "priming" refers to a process wherein a T cell has its first contact with its specific antigen and causes differentiation into effector T cells.

The term "clonal expansion" or "expansion" refers to a process wherein a specific entity is multiplied. In the context of the present disclosure, the term is preferably used in the context of an immunological response in which lymphocytes are stimulated by an antigen, proliferate, and the specific lymphocyte recognizing said antigen is amplified. Preferably, clonal expansion leads to differentiation of the lymphocytes.

The term "antigen" relates to an agent comprising an epitope against which an immune response can be generated. The term "antigen" includes, in particular, proteins and peptides. In one embodiment, an antigen is present on the surface of cells of the immune system such as antigen presenting cells like dendritic cells or macrophages. An antigen or a processing product thereof such as a T cell epitope is in one embodiment bound by a CAR molecule. Accordingly, an antigen or a processing product thereof may react specifically with T-lymphocytes (T cells). In one embodiment, an antigen is a disease-associated antigen, such as a tumor antigen, a viral antigen, or a bacterial antigen and an epitope is derived from such antigen.

The term "disease-associated antigen" is used in its broadest sense to refer to any antigen associated with a disease. A disease-associated antigen is a molecule which contains epitopes that will stimulate a host's immune system to make a cellular antigen-specific immune response and/or a humoral antibody response against the disease. The disease-associated antigen or an epitope thereof may therefore be used for therapeutic purposes. Disease-associated antigens may be associated with infection by microbes, typically microbial antigens, or associated with cancer, typically tumors.

The term "tumor antigen" refers to a constituent of cancer cells which may be derived from the cytoplasm, the cell surface and the cell nucleus. In particular, it refers to those antigens which are produced intracellularly or as surface antigens on tumor cells. A tumor antigen is typically expressed preferentially by cancer cells (e.g., it is expressed at higher levels in cancer cells than on non-cancer cells) and in some instances it is expressed solely by cancer cells. Examples of tumor antigens include, without limitation, p53, ART-4, BAGE, beta-catenin/m, Bcr-abL CAMEL, CAP-1 , CASP-8, CDC27/m, CDK4/m, CEA, the cell surface proteins of the claudin family, such as CLAUDIN-6, CLAUDIN-18.2 and CLAUDIN-12, c-MYC, CT, Cyp-B, DAM, ELF2M, ETV6-AML1, G250, GAGE, GnT-V, Gap 100, HAGE, HER-2/neu, HPV-E7, HPV-E6, HAST-2, hTERT (or hTRT), LAGE, LDLR/FUT, MAGE-A, preferably MAGE-A1 , MAGE-A2, MAGE- A3, MAGE-A4, MAGE- A5, MAGE-A6, MAGE-A7, MAGE-A8, MAGE-A9, MAGE-A 10, MAGE-A 1 1, or MAGE-A12, MAGE-B, MAGE-C, MART- 1 /Melan-A, MC1R, Myosin/m, MUC1 , MUM-1 , MUM -2, MUM -3, NA88-A, NF1 , NY-ESO-1 , NY-BR-1 , pl90 minor BCR-abL, Pml/RARa, PRAME, proteinase 3, PSA, PSM, RAGE, RU1 or RU2, SAGE, SART-1 or SART-3, SCGB3A2, SCP1 , SCP2, SCP3, SSX, SURVIVIN, TEL/AML1 , TPI/m, TRP-1 , TRP-2, TRP-2/INT2, TPTE, WT, and WT-1.

The term "viral antigen" refers to any viral component having antigenic properties, i.e. being able to provoke an immune response in an individual. The viral antigen may be a viral ribonucleoprotein or an envelope protein.

The term "bacterial antigen" refers to any bacterial component having antigenic properties, i.e. being able to provoke an immune response in an individual. The bacterial antigen may be derived from the cell wall or cytoplasm membrane of the bacterium.

The term "epitope" refers to a part or fragment a molecule such as an antigen that is recognized by the immune system. For example, the epitope may be recognized by T cells, B cells or antibodies. An epitope of an antigen may include a continuous or discontinuous portion of the antigen and may be between about 5 and about 100, such as between about 5 and about 50, more preferably between about 8 and about 30, most preferably between about 10 and about 25 amino acids in length, for example, the epitope may be preferably 9, 10, 11, 12, 13, 14, 15, 16, 17, 18, 19, 20, 21, 22, 23, 24, or 25 amino acids in length. In one embodiment, an epitope is between about 10 and about 25 amino acids in length. The term "epitope" includes T cell epitopes.

The term "T cell epitope" refers to a part or fragment of a protein that is recognized by a T cell when presented in the context of MHC molecules. The term "major histocompatibility complex" and the abbreviation "MHC" includes MHC class I and MHC class II molecules and relates to a complex of genes which is present in all vertebrates. MHC proteins or molecules are important for signaling between lymphocytes and antigen presenting cells or diseased cells in immune reactions, wherein the MHC proteins or molecules bind peptide epitopes and present them for recognition by T cell receptors on T cells. The proteins encoded by the MHC are expressed on the surface of cells, and display both self-antigens (peptide fragments from the cell itself) and non-self-antigens (e.g., fragments of invading microorganisms) to a T cell. In the case of class I MHC/peptide complexes, the binding peptides are typically about 8 to about 10 amino acids long although longer or shorter peptides may be effective. In the case of class II MHC/peptide complexes, the binding peptides are typically about 10 to about 25 amino acids long and are in particular about 13 to about 18 amino acids long, whereas longer and shorter peptides may be effective.

In one embodiment, the target antigen is a tumor antigen and the vaccine antigen or a fragment thereof (e.g., an epitope) is derived from the tumor antigen. The tumor antigen may be a "standard" antigen, which is generally known to be expressed in various cancers. The tumor antigen may also be a "neo-antigen", which is specific to an individual's tumor and has not been previously recognized by the immune system. A neo-antigen or neo-epitope may result from one or more cancer-specific mutations in the genome of cancer cells resulting in amino acid changes. If the tumor antigen is a neo-antigen, the vaccine antigen preferably comprises an epitope or a fragment of said neo-antigen comprising one or more amino acid changes.

The peptide and protein antigen can be 2-100 amino acids, including for example, 5 amino acids, 10 amino acids, 15 amino acids, 20 amino acids, 25 amino acids, 30 amino acids, 35 amino acids, 40 amino acids, 45 amino acids, or 50 amino acids in length. In some embodiments, a peptide can be greater than 50 amino acids. In some embodiments, the peptide can be greater than 100 amino acids.

According to the invention, the antigen or variant thereof should be recognizable by a CAR. Preferably, the antigen or variant thereof if recognized by a CAR is able to induce in the presence of appropriate co-stimulatory signals, stimulation, priming and/or expansion of the T cell carrying the CAR recognizing the antigen or variant thereof. In the context of the embodiments of the present invention, the antigen or variant thereof is preferably present on the surface of a cell, preferably an antigen presenting cell. Recognition of the antigen on the surface of a diseased cell may result in an immune reaction against the antigen (or cell expressing the antigen).

According to the various aspects of the invention, the aim is preferably to provide an immune response against cancer cells expressing a tumor antigen such as CLDN6 or CLDN18.2 and to treat a cancer disease involving cells expressing a tumor antigen such as CLDN6 or CLDN18.2. Preferably the invention involves the administration of CAR-engineered T cells targeted against cancer cells expressing a tumor antigen such as CLDN6 or CLDN18.2.

"Cell surface" is used in accordance with its normal meaning in the art, and thus includes the outside of the cell which is accessible to binding by proteins and other molecules. An antigen is expressed on the surface of cells if it is located at the surface of said cells and is accessible to binding by e.g. antigen-specific antibodies added to the cells. In one embodiment, an antigen expressed on the surface of cells is an integral membrane protein having an extracellular portion recognized by a CAR.

The term "extracellular portion" or "exodomain" in the context of the present invention refers to a part of a molecule such as a protein that is facing the extracellular space of a cell and preferably is accessible from the outside of said cell, e.g., by binding molecules such as antibodies located outside the cell. Preferably, the term refers to one or more extracellular loops or domains or a fragment thereof.

In one embodiment of all aspects of the invention, an antigen is expressed in a diseased cell such as a cancer cell. In one embodiment, an antigen is expressed on the surface of a diseased cell such as a cancer cell. In one embodiment, a CAR binds to an extracellular domain or to an epitope in an extracellular domain of an antigen or a variant thereof. In one embodiment, a CAR binds to native epitopes of an antigen or a variant thereof present on the surface of living cells. In one embodiment said antigen is a claudin, in particular claudin 6 or claudin 18.2, and said CAR binds to the first extracellular loop of said claudin. In one embodiment, binding of said CAR when expressed by T cells and/or present on T cells to an antigen or a variant thereof present on cells such as antigen presenting cells results in stimulation, priming and/or expansion of said T cells. In one embodiment, binding of said CAR when expressed by T cells and/or present on T cells to an antigen present on diseased cells such as cancer cells results in cytolysis and/or apoptosis of the diseased cells, wherein said T cells preferably release cytotoxic factors, e.g. perforins and granzymes.

### Immune checkpoint inhibitors

In certain embodiments, immune checkpoint inhibitors are used in combination with other therapeutic agents described herein.

As used herein, "immune checkpoint" refers to co-stimulatory and inhibitory signals that regulate the amplitude and quality of T cell receptor recognition of an antigen. In certain embodiments, the immune checkpoint is an inhibitory signal. In certain embodiments, the inhibitory signal is the interaction between PD-1 and PD-L1. In certain embodiments, the inhibitory signal is the interaction between CTLA-4 and CD80 or CD86 to displace CD28 binding. In certain embodiments the inhibitory signal is the interaction between LAG3 and MHC class II molecules. In certain embodiments, the inhibitory signal is the interaction between TIM3 and galectin 9.

As used herein, "immune checkpoint inhibitor" refers to a molecule that totally or partially reduces, inhibits, interferes with or modulates one or more checkpoint proteins. In certain embodiments, the immune checkpoint inhibitor prevents inhibitory signals associated with the immune checkpoint. In certain embodiments, the immune checkpoint inhibitor is an antibody, or fragment thereof that disrupts inhibitory signaling associated with the immune checkpoint. In certain embodiments, the immune checkpoint inhibitor is a small molecule that disrupts inhibitory signaling. In certain embodiments, the immune checkpoint inhibitor is an antibody, fragment thereof, or antibody mimic, that prevents the interaction between checkpoint blocker proteins, e.g., an antibody, or fragment thereof, that prevents the interaction between PD-1 and PD-L1. In certain embodiments, the immune checkpoint inhibitor is an antibody, or fragment thereof, that prevents the interaction between CTLA-4 and CD80 or CD86. In certain embodiments, the immune checkpoint inhibitor is an antibody, or fragment thereof, that prevents the interaction between LAG3 and its ligands, or TIM-3 and its ligands. The checkpoint inhibitor may also be in the form of the soluble form of the molecules (or variants thereof) themselves, e.g., a soluble PD-L1 or PD-L1 fusion.

The "Programmed Death-1 (PD-1)" receptor refers to an immuno-inhibitory receptor belonging to the CD28 family. PD-1 is expressed predominantly on previously activated T cells *in vivo,* and binds to two ligands, PD-L1 and PD-L2. The term "PD-1" as used herein includes human PD-1 (hPD-1), variants, isoforms, and species homologs of hPD-1, and analogs having at least one common epitope with hPD-1.

"Programmed Death Ligand-1 (PD-L1)" is one of two cell surface glycoprotein ligands for PD-1 (the other being PD-L2) that downregulates T cell activation and cytokine secretion upon binding to PD-1. The term "PD-L1" as used herein includes human PD-L1 (hPD-L1), variants, isoforms, and species homologs of hPD-L1, and analogs having at least one common epitope with hPD-L1.

"Cytotoxic T Lymphocyte Associated Antigen-4 (CTLA-4)" is a T cell surface molecule and is a member of the immunoglobulin superfamily. This protein downregulates the immune system by binding to CD80 and CD86. The term "CTLA-4" as used herein includes human CTLA-4 (hCTLA-4), variants, isoforms, and species homologs of hCTLA-4, and analogs having at least one common epitope with hCTLA-4.

"Lymphocyte Activation Gene-3 (LAG3)" is an inhibitory receptor associated with inhibition of lymphocyte activity by binding to MHC class II molecules. This receptor enhances the function of Treg cells and inhibits CD8+ effector T cell function. The term "LAG3" as used herein includes human LAG3 (hLAG3), variants, isoforms, and species homologs of hLAG3, and analogs having at least one common epitope.

"T Cell Membrane Protein-3 (TIM3)" is an inhibitory receptor involved in the inhibition of lymphocyte activity by inhibition of TH1 cells responses. Its ligand is galectin 9, which is upregulated in various types of cancers. The term "TIM3" as used herein includes human TIM3 (hTIM3), variants, isoforms, and species homologs of hTIM3, and analogs having at least one common epitope.

The "B7 family" refers to inhibitory ligands with undefined receptors. The B7 family encompasses B7-H3 and B7-H4, both upregulated on tumor cells and tumor infiltrating cells.

In certain embodiments, the immune checkpoint inhibitor suitable for use in the methods disclosed herein, is an antagonist of inhibitory signals, e.g., an antibody which targets, for example, PD-1, PD-L1, CTLA-4, LAG3, B7-H3, B7-H4, or TIM3. These ligands and receptors are reviewed in Pardoll, D., Nature. 12: 252-264, 2012.

In certain embodiments, the immune checkpoint inhibitor is an antibody or an antigen-binding portion thereof, that disrupts or inhibits signaling from an inhibitory immunoregulator. In certain embodiments, the immune checkpoint inhibitor is a small molecule that disrupts or inhibits signaling from an inhibitory immunoregulator.

In certain embodiments, the inhibitory immunoregulator is a component of the PD-1/PD-L1 signaling pathway. Accordingly, certain embodiments of the disclosure provide for administering to a subject an antibody or an antigen-binding portion thereof that disrupts the interaction between the PD-1 receptor and its ligand, PD-L1. Antibodies which bind to PD-1 and disrupt the interaction between the PD-1 and its ligand, PD-L1, are known in the art. In certain embodiments, the antibody or antigen-binding portion thereof binds specifically to PD-1. In certain embodiments, the antibody or antigen-binding portion thereof binds specifically to PD-L1 and inhibits its interaction with PD-1, thereby increasing immune activity.

In certain embodiments, the inhibitory immunoregulator is a component of the CTLA4 signaling pathway. Accordingly, certain embodiments of the disclosure provide for administering to a subject an antibody or an antigen-binding portion thereof that targets CTLA4 and disrupts its interaction with CD80 and CD86.

In certain embodiments, the inhibitory immunoregulator is a component of the LAG3 (lymphocyte activation gene 3) signaling pathway. Accordingly, certain embodiments of the disclosure provide for administering to a subject an antibody or an antigen-binding portion thereof that targets LAG3 and disrupts its interaction with MHC class II molecules.

In certain embodiments, the inhibitory immunoregulator is a component of the B7 family signaling pathway. In certain embodiments, the B7 family members are B7-H3 and B7-H4, Accordingly, certain embodiments of the disclosure provide for administering to a subject an antibody or an antigen-binding portion thereof that targets B7-H3 or H4. The B7 family does not have any defined receptors but these ligands are upregulated on tumor cells or tumor-infiltrating cells. Preclinical mouse models have shown that blockade of these ligands can enhance anti-tumor immunity.

In certain embodiments, the inhibitory immunoregulator is a component of the TIM3 (T cell membrane protein 3) signaling pathway. Accordingly, certain embodiments of the disclosure provide for administering to a subject an antibody or an antigen-binding portion thereof that targets TIM3 and disrupts its interaction with galectin 9.

It will be understood by one of ordinary skill in the art that other immune checkpoint targets can also be targeted by antagonists or antibodies, provided that the targeting results in the stimulation of an immune response such as an anti-tumor immune response as reflected in, e.g., an increase in T cell proliferation, enhanced T cell activation, and/or increased cytokine production (e.g., IFN-y, IL2).

According to the disclosure, the term "antibody" refers to a glycoprotein comprising at least two heavy (H) chains and two light (L) chains inter-connected by disulfide bonds. The term "antibody" includes monoclonal antibodies, recombinant antibodies, human antibodies, humanized antibodies and chimeric antibodies. Each heavy chain is comprised of a heavy chain variable region (abbreviated herein as VH) and a heavy chain constant region. Each light chain is comprised of a light chain variable region (abbreviated herein as VL) and a light chain constant region. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

Antibodies may be derived from different species, including but not limited to mouse, rat, rabbit, guinea pig and human.

Antibodies described herein include IgA such as lgA1 or IgA2, IgG1, IgG2, IgG3, IgG4, IgE, IgM, and IgD antibodies. In various embodiments, the antibody is an lgG1 antibody, more particularly an IgG1, kappa or IgG1, lambda isotype (i.e. IgG1, κ, λ), an IgG2a antibody (e.g. IgG2a, κ, λ), an IgG2b antibody (e.g. IgG2b, κ, λ), an IgG3 antibody (e.g. IgG3, κ, λ) or an lgG4 antibody (e.g. IgG4, κ, λ).

The terms "antigen-binding portion" of an antibody (or simply "binding portion") or "antigen-binding fragment" of an antibody (or simply "binding fragment") or similar terms refer to one or more fragments of an antibody that retain the ability to specifically bind to an antigen. It has been shown that the antigen-binding function of an antibody can be performed by fragments of a full-length antibody. Examples of binding fragments encompassed within the term "antigen-binding portion" of an antibody include (i) Fab fragments, monovalent fragments consisting of the VL, VH, CL and CH domains; (ii) F(ab')₂ fragments, bivalent fragments comprising two Fab fragments linked by a disulfide bridge at the hinge region; (iii) Fd fragments consisting of the VH and CH domains; (iv) Fv fragments consisting of the VL and VH domains of a single arm of an antibody, (v) dAb fragments (Ward et al., (1989) Nature 341: 544-546), which consist of a VH domain; (vi) isolated complementarity determining regions (CDR), and (vii) combinations of two or more isolated CDRs which may optionally be joined by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242: 423-426; and Huston et al. (1988) Proc. Natl. Acad. Sci. USA 85: 5879-5883). Such single chain antibodies are also intended to be encompassed within the term "antigen-binding fragment" of an antibody. A further example is binding-domain immunoglobulin fusion proteins comprising (i) a binding domain polypeptide that is fused to an immunoglobulin hinge region polypeptide, (ii) an immunoglobulin heavy chain CH2 constant region fused to the hinge region, and (iii) an immunoglobulin heavy chain CH3 constant region fused to the CH2 constant region. The binding domain polypeptide can be a heavy chain variable region or a light chain variable region. The binding-domain immunoglobulin fusion proteins are further disclosed in US 2003/0118592 and US 2003/0133939. These antibody fragments are obtained using conventional techniques known to those with skill in the art, and the fragments are screened for utility in the same manner as are intact antibodies.

### RNA Targeting

According to the disclosure, after administration of the RNA described herein, at least a portion of the RNA is delivered to a target cell. In one embodiment, at least a portion of the RNA is delivered to the cytosol of the target cell. In one embodiment, the RNA is translated by the target cell to produce the encoded peptide or protein.

Some aspects of the disclosure involve the targeted delivery of the RNA disclosed herein (e.g., RNA encoding cytokine and RNA encoding an antigen or a variant thereof).

In one embodiment, the disclosure involves targeting the lymphatic system, in particular secondary lymphoid organs, more specifically spleen. Targeting the lymphatic system, in particular secondary lymphoid organs, more specifically spleen is in particular preferred if the RNA administered is RNA encoding an antigen or a variant thereof.

In one embodiment, the target cell is a spleen cell. In one embodiment, the target cell is an antigen presenting cell such as a professional antigen presenting cell in the spleen. In one embodiment, the target cell is a dendritic cell in the spleen.

The "lymphatic system" is part of the circulatory system and an important part of the immune system, comprising a network of lymphatic vessels that carry lymph. The lymphatic system consists of lymphatic organs, a conducting network of lymphatic vessels, and the circulating lymph. The primary or central lymphoid organs generate lymphocytes from immature progenitor cells. The thymus and the bone marrow constitute the primary lymphoid organs. Secondary or peripheral lymphoid organs, which include lymph nodes and the spleen, maintain mature naive lymphocytes and initiate an adaptive immune response.

RNA may be delivered to spleen by so-called lipoplex formulations, in which the RNA is bound to liposomes comprising a cationic lipid and optionally an additional or helper lipid to form injectable nanoparticle formulations. The liposomes may be obtained by injecting a solution of the lipids in ethanol into water or a suitable aqueous phase. RNA lipoplex particles may be prepared by mixing the liposomes with RNA. Spleen targeting RNA lipoplex particles are described in WO 2013/143683, herein incorporated by reference. It has been found that RNA lipoplex particles having a net negative charge may be used to preferentially target spleen tissue or spleen cells such as antigen-presenting cells, in particular dendritic cells. Accordingly, following administration of the RNA lipoplex particles, RNA accumulation and/or RNA expression in the spleen occurs. Thus, RNA lipoplex particles of the disclosure may be used for expressing RNA in the spleen. In an embodiment, after administration of the RNA lipoplex particles, no or essentially no RNA accumulation and/or RNA expression in the lung and/or liver occurs. In one embodiment, after administration of the RNA lipoplex particles, RNA accumulation and/or RNA expression in antigen presenting cells, such as professional antigen presenting cells in the spleen occurs. Thus, RNA lipoplex particles of the disclosure may be used for expressing RNA in such antigen presenting cells. In one embodiment, the antigen presenting cells are dendritic cells and/or macrophages.

In the context of the present disclosure, the term "RNA lipoplex particle" relates to a particle that contains lipid, in particular cationic lipid, and RNA. Electrostatic interactions between positively charged liposomes and negatively charged RNA results in complexation and spontaneous formation of RNA lipoplex particles. Positively charged liposomes may be generally synthesized using a cationic lipid, such as DOTMA, and additional lipids, such as DOPE. In one embodiment, a RNA lipoplex particle is a nanoparticle.

As used herein, a "cationic lipid" refers to a lipid having a net positive charge. Cationic lipids bind negatively charged RNA by electrostatic interaction to the lipid matrix. Generally, cationic lipids possess a lipophilic moiety, such as a sterol, an acyl or diacyl chain, and the head group of the lipid typically carries the positive charge. Examples of cationic lipids include, but are not limited to 1,2-di-O-octadecenyl-3-trimethylammonium propane (DOTMA), dimethyldioctadecylammonium (DDAB); 1,2-dioleoyl-3-trimethylammonium propane (DOTAP); 1,2-dioleoyl-3-dimethylammonium-propane (DODAP); 1,2-diacyloxy-3-dimethylammonium propanes; 1,2-dialkyloxy-3- dimethylammonium propanes; dioctadecyldimethyl ammonium chloride (DODAC), 2,3-di(tetradecoxy)propyl-(2-hydroxyethyl)-dimethylazanium (DMRIE), 1,2-dimyristoyl-sn-glycero-3-ethylphosphocholine (DMEPC), l,2-dimyristoyl-3-trimethylammonium propane (DMTAP), 1,2-dioleyloxypropyl-3-dimethyl-hydroxyethyl ammonium bromide (DORIE), and 2,3-dioleoyloxy- N-[2(spermine carboxamide)ethyl]-N,N-dimethyl-I-propanamium trifluoroacetate (DOSPA). Preferred are DOTMA, DOTAP, DODAC, and DOSPA. In specific embodiments, the cationic lipid is DOTMA and/or DOTAP.

An additional lipid may be incorporated to adjust the overall positive to negative charge ratio and physical stability of the RNA lipoplex particles. In certain embodiments, the additional lipid is a neutral lipid. As used herein, a "neutral lipid" refers to a lipid having a net charge of zero. Examples of neutral lipids include, but are not limited to, 1,2-di-(9Z-octadecenoyl)-sn-glycero-3-phosphoethanolamine (DOPE), 1,2-dioleoyl-sn-glycero-3-phosphocholine (DOPC), diacylphosphatidyl choline, diacylphosphatidyl ethanol amine, ceramide, sphingoemyelin, cephalin, cholesterol, and cerebroside. In specific embodiments, the additional lipid is DOPE, cholesterol and/or DOPC.

In certain embodiments, the RNA lipoplex particles include both a cationic lipid and an additional lipid. In an exemplary embodiment, the cationic lipid is DOTMA and the additional lipid is DOPE.

In some embodiments, the molar ratio of the at least one cationic lipid to the at least one additional lipid is from about 10:0 to about 1:9, about 4:1 to about 1:2, or about 3:1 to about 1:1. In specific embodiments, the molar ratio may be about 3:1, about 2.75:1, about 2.5:1, about 2.25:1, about 2:1, about 1.75:1, about 1.5:1, about 1.25:1, or about 1:1. In an exemplary embodiment, the molar ratio of the at least one cationic lipid to the at least one additional lipid is about 2:1.

RNA lipoplex particles described herein have an average diameter that in one embodiment ranges from about 200 nm to about 1000 nm, from about 200 nm to about 800 nm, from about 250 to about 700 nm, from about 400 to about 600 nm, from about 300 nm to about 500 nm, or from about 350 nm to about 400 nm. In specific embodiments, the RNA lipoplex particles have an average diameter of about 200 nm, about 225 nm, about 250 nm, about 275 nm, about 300 nm, about 325 nm, about 350 nm, about 375 nm, about 400 nm, about 425 nm, about 450 nm, about 475 nm, about 500 nm, about 525 nm, about 550 nm, about 575 nm, about 600 nm, about 625 nm, about 650 nm, about 700 nm, about 725 nm, about 750 nm, about 775 nm, about 800 nm, about 825 nm, about 850 nm, about 875 nm, about 900 nm, about 925 nm, about 950 nm, about 975 nm, or about 1000 nm. In an embodiment, the RNA lipoplex particles have an average diameter that ranges from about 250 nm to about 700 nm. In another embodiment, the RNA lipoplex particles have an average diameter that ranges from about 300 nm to about 500 nm. In an exemplary embodiment, the RNA lipoplex particles have an average diameter of about 400 nm.

The electric charge of the RNA lipoplex particles of the present disclosure is the sum of the electric charges present in the at least one cationic lipid and the electric charges present in the RNA. The charge ratio is the ratio of the positive charges present in the at least one cationic lipid to the negative charges present in the RNA. The charge ratio of the positive charges present in the at least one cationic lipid to the negative charges present in the RNA is calculated by the following equation: charge ratio=[(cationic lipid concentration (mol)) * (the total number of positive charges in the cationic lipid)] / [(RNA concentration (mol)) * (the total number of negative charges in RNA)].

The spleen targeting RNA lipoplex particles described herein at physiological pH preferably have a net negative charge such as a charge ratio of positive charges to negative charges from about 1.9:2 to about 1:2. In specific embodiments, the charge ratio of positive charges to negative charges in the RNA lipoplex particles at physiological pH is about 1.9:2.0, about 1.8:2.0, about 1.7:2.0, about 1.6:2.0, about 1.5:2.0, about 1.4:2.0, about 1.3:2.0, about 1.2:2.0, about 1.1:2.0, or about 1:2.0.

Cytokines such as extended-PK cytokines, in particular extended-PK interleukins, such as those described herein may be delivered to a target organ or target tissue in a subject comprising administering to the subject RNA encoding a cytokine in a formulation for preferential delivery of RNA to said target organ or tissue.

In one embodiment, the target organ is the lymphatic system, in particular secondary lymphoid organs, more specifically spleen, and the target tissue is tissue of the lymphatic system, in particular tissue of secondary lymphoid organs, more specifically spleen tissue. The delivery of a cytokine to such target tissue is preferred, in particular, if presence of the cytokine in this organ or tissue is desired (e.g., for inducing an immune response, in particular in case cytokines are required during T cell priming or for activation of resident immune cells), while it is not desired that the cytokine is present systemically, in particular in significant amounts (e.g., because the cytokine has systemic toxicity). Particularly preferred examples of suitable cytokines are cytokines involved in T cell priming.

In another embodiment of delivering a cytokine to a target organ or target tissue in a subject, the target organ is liver and the target tissue is liver tissue. The delivery of a cytokine to such target tissue is preferred, in particular, if presence of the cytokine in this organ or tissue is desired and/or if it is desired to express large amounts of the cytokine and/or if systemic presence of the cytokine, in particular in significant amounts, is desired or required.

In one embodiment, the RNA encoding a cytokine is administered in a formulation for targeting liver. Such formulations are described herein. Examples of suitable cytokines include IL2, IL7 or IL21, fragments and variants thereof, and fusion proteins of these cytokines, fragments and variants, such as extended-PK cytokines, such as those described herein. Particularly preferred examples of suitable cytokines are cytokines involved in T cell proliferation and/or maintenance.

RNA delivery systems have an inherent preference to the liver. This pertains to lipid-based particles, cationic and neutral nanoparticles, in particular lipid nanoparticles such as liposomes, nanomicelles and lipophilic ligands in bioconjugates. Liver accumulation is caused by the discontinuous nature of the hepatic vasculature or the lipid metabolism (liposomes and lipid or cholesterol conjugates).

For *in vivo* delivery of RNA to the liver, a drug delivery system may be used to transport the RNA into the liver by preventing its degradation. For example, polyplex nanomicelles consisting of a poly(ethylene glycol) (PEG)-coated surface and an mRNA-containing core is a useful system because the nanomicelles provide excellent *in vivo* stability of the RNA, under physiological conditions. Furthermore, the stealth property provided by the polyplex nanomicelle surface, composed of dense PEG palisades, effectively evades host immune defenses.

### Pharmaceutical compositions

The agents described herein may be administered in pharmaceutical compositions or medicaments and may be administered in the form of any suitable pharmaceutical composition.

In one embodiment of all aspects of the invention, the components described herein such as T cells genetically modified to express a CAR, nucleic acid encoding a cytokine or nucleic acid encoding an antigen or a variant thereof either together or separate from each other may be administered in a pharmaceutical composition which may comprise a pharmaceutically acceptable carrier and may optionally comprise one or more adjuvants, stabilizers etc. In one embodiment, the pharmaceutical composition is for therapeutic or prophylactic treatments, e.g., for use in treating or preventing a disease involving an antigen such as a cancer disease such as those described herein.

The term "pharmaceutical composition" relates to a formulation comprising a therapeutically effective agent, preferably together with pharmaceutically acceptable carriers, diluents and/or excipients. Said pharmaceutical composition is useful for treating, preventing, or reducing the severity of a disease or disorder by administration of said pharmaceutical composition to a subject. A pharmaceutical composition is also known in the art as a pharmaceutical formulation.

The pharmaceutical compositions of the present disclosure preferably comprise one or more adjuvants or may be administered with one or more adjuvants. The term "adjuvant" relates to a compound which prolongs, enhances or accelerates an immune response. Adjuvants comprise a heterogeneous group of compounds such as oil emulsions (e.g., Freund's adjuvants), mineral compounds (such as alum), bacterial products (such as Bordetella pertussis toxin), or immune-stimulating complexes. Examples of adjuvants include, without limitation, LPS, GP96, CpG oligodeoxynucleotides, growth factors, and cyctokines, such as monokines, lymphokines, interleukins, chemokines. The chemokines may be IL1, IL2, IL3, IL4, IL5, IL6, IL7, IL8, IL9, IL10, IL12, IFNα, IFNγ, GM-CSF, LT-a. Further known adjuvants are aluminium hydroxide, Freund's adjuvant or oil such as Montanide^{®} ISA51. Other suitable adjuvants for use in the present disclosure include lipopeptides, such as Pam3Cys.

The pharmaceutical compositions according to the present disclosure are generally applied in a "pharmaceutically effective amount" and in "a pharmaceutically acceptable preparation".

The term "pharmaceutically acceptable" refers to the non-toxicity of a material which does not interact with the action of the active component of the pharmaceutical composition.

The term "pharmaceutically effective amount" or "therapeutically effective amount" refers to the amount which achieves a desired reaction or a desired effect alone or together with further doses. In the case of the treatment of a particular disease, the desired reaction preferably relates to inhibition of the course of the disease. This comprises slowing down the progress of the disease and, in particular, interrupting or reversing the progress of the disease. The desired reaction in a treatment of a disease may also be delay of the onset or a prevention of the onset of said disease or said condition. An effective amount of the compositions described herein will depend on the condition to be treated, the severeness of the disease, the individual parameters of the patient, including age, physiological condition, size and weight, the duration of treatment, the type of an accompanying therapy (if present), the specific route of administration and similar factors. Accordingly, the doses administered of the compositions described herein may depend on various of such parameters. In the case that a reaction in a patient is insufficient with an initial dose, higher doses (or effectively higher doses achieved by a different, more localized route of administration) may be used.

The pharmaceutical compositions of the present disclosure may contain salts, buffers, preservatives, and optionally other therapeutic agents. In one embodiment, the pharmaceutical compositions of the present disclosure comprise one or more pharmaceutically acceptable carriers, diluents and/or excipients.

Suitable preservatives for use in the pharmaceutical compositions of the present disclosure include, without limitation, benzalkonium chloride, chlorobutanol, paraben and thimerosal.

The term "excipient" as used herein refers to a substance which may be present in a pharmaceutical composition of the present disclosure but is not an active ingredient. Examples of excipients, include without limitation, carriers, binders, diluents, lubricants, thickeners, surface active agents, preservatives, stabilizers, emulsifiers, buffers, flavoring agents, or colorants.

The term "diluent" relates a diluting and/or thinning agent. Moreover, the term "diluent" includes any one or more of fluid, liquid or solid suspension and/or mixing media. Examples of suitable diluents include ethanol, glycerol and water.

The term "carrier" refers to a component which may be natural, synthetic, organic, inorganic in which the active component is combined in order to facilitate, enhance or enable administration of the pharmaceutical composition. A carrier as used herein may be one or more compatible solid or liquid fillers, diluents or encapsulating substances, which are suitable for administration to subject. Suitable carrier include, without limitation, sterile water, Ringer, Ringer lactate, sterile sodium chloride solution, isotonic saline, polyalkylene glycols, hydrogenated naphthalenes and, in particular, biocompatible lactide polymers, lactide/glycolide copolymers or polyoxyethylene/polyoxy-propylene copolymers. In one embodiment, the pharmaceutical composition of the present disclosure includes isotonic saline.

Pharmaceutically acceptable carriers, excipients or diluents for therapeutic use are well known in the pharmaceutical art, and are described, for example, in Remington's Pharmaceutical Sciences, Mack Publishing Co. (A. R Gennaro edit. 1985).

Pharmaceutical carriers, excipients or diluents can be selected with regard to the intended route of administration and standard pharmaceutical practice.

In one embodiment, pharmaceutical compositions described herein may be administered intravenously, intraarterially, subcutaneously, intradermally or intramuscularly. In certain embodiments, the pharmaceutical composition is formulated for local administration or systemic administration. Systemic administration may include enteral administration, which involves absorption through the gastrointestinal tract, or parenteral administration. As used herein, "parenteral administration" refers to the administration in any manner other than through the gastrointestinal tract, such as by intravenous injection. In a preferred embodiment, the pharmaceutical compositions is formulated for systemic administration. In another preferred embodiment, the systemic administration is by intravenous administration.

In one embodiment of all aspects of the invention, nucleic acid encoding a cytokine or encoding an antigen or variant thereof is administered systemically. In one embodiment of all aspects of the invention, after systemic administration of the nucleic acid encoding the antigen or variant thereof, expression of the antigen or variant thereof in spleen occurs. In one embodiment of all aspects of the invention, after systemic administration of the nucleic acid encoding the antigen or variant thereof, expression of the antigen or variant thereof in antigen presenting cells, preferably professional antigen presenting cells occurs. In one embodiment, the antigen presenting cells are selected from the group consisting of dendritic cells, macrophages and B cells. In one embodiment of all aspects of the invention, after systemic administration of the nucleic acid encoding the antigen or variant thereof, no or essentially no expression of the antigen or variant thereof in lung and/or liver occurs. In one embodiment of all aspects of the invention, after systemic administration of the nucleic acid encoding the antigen or variant thereof, expression of the antigen or variant thereof in spleen is at least 5-fold the amount of expression in lung.

The term "co-administering" as used herein means a process whereby different compounds or compositions (e.g., RNA encoding an interleukin and RNA encoding an antigen or a variant thereof) are administered to the same patient simultaneously, at essentially the same time, or sequentially. If administration takes place simultaneously the different compounds or compositions need not be administered within the same composition.

### Treatments

The agents, compositions and methods described herein can be used to treat a subject with a disease, e.g., a disease characterized by the presence of diseased cells expressing an antigen. Particularly preferred diseases are cancer diseases. For example, if the antigen is derived from a virus, the agents, compositions and methods may be useful in the treatment of a viral disease caused by said virus. If the antigen is a tumor antigen, the agents, compositions and methods may be useful in the treatment of a cancer disease wherein cancer cells express said tumor antigen.

In one embodiment, the present disclosure relates to a method for inducing an immune response in a subject. In an exemplary embodiment, the immune response is against cancer.

The term "disease" refers to an abnormal condition that affects the body of an individual. A disease is often construed as a medical condition associated with specific symptoms and signs. A disease may be caused by factors originally from an external source, such as infectious disease, or it may be caused by internal dysfunctions, such as autoimmune diseases. In humans, "disease" is often used more broadly to refer to any condition that causes pain, dysfunction, distress, social problems, or death to the individual afflicted, or similar problems for those in contact with the individual. In this broader sense, it sometimes includes injuries, disabilities, disorders, syndromes, infections, isolated symptoms, deviant behaviors, and atypical variations of structure and function, while in other contexts and for other purposes these may be considered distinguishable categories. Diseases usually affect individuals not only physically, but also emotionally, as contracting and living with many diseases can alter one's perspective on life, and one's personality.

In the present context, the term "treatment", "treating" or "therapeutic intervention" relates to the management and care of a subject for the purpose of combating a condition such as a disease or disorder. The term is intended to include the full spectrum of treatments for a given condition from which the subject is suffering, such as administration of the therapeutically effective compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of an individual for the purpose of combating the disease, condition or disorder and includes the administration of the active compounds to prevent the onset of the symptoms or complications.

The term "therapeutic treatment" relates to any treatment which improves the health status and/or prolongs (increases) the lifespan of an individual. Said treatment may eliminate the disease in an individual, arrest or slow the development of a disease in an individual, inhibit or slow the development of a disease in an individual, decrease the frequency or severity of symptoms in an individual, and/or decrease the recurrence in an individual who currently has or who previously has had a disease.

The terms "prophylactic treatment" or "preventive treatment" relate to any treatment that is intended to prevent a disease from occurring in an individual. The terms "prophylactic treatment" or "preventive treatment" are used herein interchangeably.

The terms "individual" and "subject" are used herein interchangeably. They refer to a human or another mammal (e.g. mouse, rat, rabbit, dog, cat, cattle, swine, sheep, horse or primate) that can be afflicted with or is susceptible to a disease or disorder (e.g., cancer) but may or may not have the disease or disorder. In many embodiments, the individual is a human being. Unless otherwise stated, the terms "individual" and "subject" do not denote a particular age, and thus encompass adults, elderlies, children, and newborns. In embodiments of the present disclosure, the "individual" or "subject" is a "patient".

The term "patient" means an individual or subject for treatment, in particular a diseased individual or subject.

In one embodiment of the disclosure, the aim is to provide an immune response against diseased cells expressing an antigen such as cancer cells expressing a tumor antigen, and to treat a disease such as a cancer disease involving cells expressing an antigen such as a tumor antigen.

An immune response against an antigen may be elicited which may be therapeutic or partially or fully protective. Pharmaceutical compositions described herein are applicable for inducing or enhancing an immune response. Pharmaceutical compositions described herein are thus useful in a prophylactic and/or therapeutic treatment of a disease involving an antigen.

As used herein, "immune response" refers to an integrated bodily response to an antigen or a cell expressing an antigen and refers to a cellular immune response and/or a humoral immune response. A cellular immune response includes, without limitation, a cellular response directed to cells expressing an antigen. Such cells may be characterized by expression of an antigen on their cell surface or by presentation of an antigen with class I or class II MHC molecule. The cellular response relates to T lymphocytes, which may be classified as helper T cells (also termed CD4+ T cells) that play a central role by regulating the immune response or killer cells (also termed cytotoxic T cells, CD8+ T cells, or CTLs) that induce apoptosis in infected cells or cancer cells. In one embodiment, administering a pharmaceutical composition of the present disclosure involves stimulation of an anti-tumor CD8+ T cell response against cancer cells expressing one or more tumor antigens.

The present disclosure contemplates an immune response that may be protective, preventive, prophylactic and/or therapeutic. As used herein, "induces [or inducing] an immune response" may indicate that no immune response against a particular antigen was present before induction or it may indicate that there was a basal level of immune response against a particular antigen before induction, which was enhanced after induction. Therefore, "induces [or inducing] an immune response" includes "enhances for enhancing] an immune response".

The term "immunotherapy" relates to the treatment of a disease or condition by inducing, or enhancing an immune response.

The term "vaccination" or "immunization" describes the process of administering an antigen to an individual with the purpose of inducing an immune response, for example, for therapeutic or prophylactic reasons.

In one embodiment, the present disclosure envisions embodiments wherein RNA formulations such as RNA particles as described herein are administered.

Accordingly, the present disclosure relates to RNA as described herein for use in a prophylactic and/or therapeutic treatment of a disease involving an antigen, preferably a cancer disease.

The term "macrophage" refers to a subgroup of phagocytic cells produced by the differentiation of monocytes. Macrophages which are activated by inflammation, immune cytokines or microbial products nonspecifically engulf and kill foreign pathogens within the macrophage by hydrolytic and oxidative attack resulting in degradation of the pathogen. Peptides from degraded proteins are displayed on the macrophage cell surface where they can be recognized by T cells, and they can directly interact with antibodies on the B cell surface, resulting in T and B cell activation and further stimulation of the immune response. Macrophages belong to the class of antigen presenting cells. In one embodiment, the macrophages are splenic macrophages.

The term "dendritic cell" (DC) refers to another subtype of phagocytic cells belonging to the class of antigen presenting cells. In one embodiment, dendritic cells are derived from hematopoietic bone marrow progenitor cells. These progenitor cells initially transform into immature dendritic cells. These immature cells are characterized by high phagocytic activity and low T cell activation potential. Immature dendritic cells constantly sample the surrounding environment for pathogens such as viruses and bacteria. Once they have come into contact with a presentable antigen, they become activated into mature dendritic cells and begin to migrate to the spleen or to the lymph node. Immature dendritic cells phagocytose pathogens and degrade their proteins into small pieces and upon maturation present those fragments at their cell surface using MHC molecules. Simultaneously, they upregulate cell-surface receptors that act as co-receptors in T cell activation such as CD80, CD86, and CD40 greatly enhancing their ability to activate T cells. They also upregulate CCR7, a chemotactic receptor that induces the dendritic cell to travel through the blood stream to the spleen or through the lymphatic system to a lymph node. Here they act as antigen-presenting cells and activate helper T cells and killer T cells as well as B cells by presenting them antigens, alongside non-antigen specific co-stimulatory signals. Thus, dendritic cells can actively induce a T cell- or B cell-related immune response. In one embodiment, the dendritic cells are splenic dendritic cells.

The term "antigen presenting cell" (APC) is a cell of a variety of cells capable of displaying, acquiring, and/or presenting at least one antigen or antigenic fragment on (or at) its cell surface. Antigen-presenting cells can be distinguished in professional antigen presenting cells and non-professional antigen presenting cells.

The term "professional antigen presenting cells" relates to antigen presenting cells which constitutively express the Major Histocompatibility Complex class II (MHC class II) molecules required for interaction with naive T cells. If a T cell interacts with the MHC class II molecule complex on the membrane of the antigen presenting cell, the antigen presenting cell produces a co-stimulatory molecule inducing activation of the T cell. Professional antigen presenting cells comprise dendritic cells and macrophages.

The term "non-professional antigen presenting cells" relates to antigen presenting cells which do not constitutively express MHC class II molecules, but upon stimulation by certain cytokines such as interferon-gamma. Exemplary, non-professional antigen presenting cells include fibroblasts, thymic epithelial cells, thyroid epithelial cells, glial cells, pancreatic beta cells or vascular endothelial cells.

"Antigen processing" refers to the degradation of an antigen into procession products, which are fragments of said antigen (e.g., the degradation of a protein into peptides) and the association of one or more of these fragments (e.g., via binding) with MHC molecules for presentation by cells, such as antigen presenting cells to specific T cells.

The term "disease involving an antigen", "disease involving cells expressing an antigen" or similar terms refer to any disease which implicates an antigen, e.g. a disease which is characterized by the presence of an antigen. The disease can be an infectious disease, or a cancer disease or simply cancer. As mentioned above, the antigen may be a disease-associated antigen, such as a tumor-associated antigen, a viral antigen, or a bacterial antigen. Preferably a disease involving an antigen is a disease involving cells expressing an antigen, preferably on the cell surface.

The term "infectious disease" refers to any disease which can be transmitted from individual to individual or from organism to organism, and is caused by a microbial agent (e.g. common cold). Infectious diseases are known in the art and include, for example, a viral disease, a bacterial disease, or a parasitic disease, which diseases are caused by a virus, a bacterium, and a parasite, respectively. In this regard, the infectious disease can be, for example, hepatitis, sexually transmitted diseases (e.g. chlamydia or gonorrhea), tuberculosis, HIV/acquired immune deficiency syndrome (AIDS), diphtheria, hepatitis B, hepatitis C, cholera, severe acute respiratory syndrome (SARS), the bird flu, and influenza.

The terms "cancer disease" or "cancer" refer to or describe the physiological condition in an individual that is typically characterized by unregulated cell growth. Examples of cancers include, but are not limited to, carcinoma, lymphoma, blastoma, sarcoma, and leukemia. More particularly, examples of such cancers include bone cancer, blood cancer lung cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, prostate cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the renal pelvis, neoplasms of the central nervous system (CNS), neuroectodermal cancer, spinal axis tumors, glioma, meningioma, and pituitary adenoma. The term "cancer" according to the disclosure also comprises cancer metastases.

Combination strategies in cancer treatment may be desirable due to a resulting synergistic effect, which may be considerably stronger than the impact of a monotherapeutic approach. In one embodiment, the pharmaceutical composition is administered with an immunotherapeutic agent. As used herein "immunotherapeutic agent" relates to any agent that may be involved in activating a specific immune response and/or immune effector function(s). The present disclosure contemplates the use of an antibody as an immunotherapeutic agent. Without wishing to be bound by theory, antibodies are capable of achieving a therapeutic effect against cancer cells through various mechanisms, including inducing apoptosis, block components of signal transduction pathways or inhibiting proliferation of tumor cells. In certain embodiments, the antibody is a monoclonal antibody. A monoclonal antibody may induce cell death via antibody-dependent cell mediated cytotoxicity (ADCC), or bind complement proteins, leading to direct cell toxicity, known as complement dependent cytotoxicity (CDC). Non-limiting examples of anti-cancer antibodies and potential antibody targets (in brackets) which may be used in combination with the present disclosure include: Abagovomab (CA-125), Abciximab (CD41), Adecatumumab (EpCAM), Afutuzumab (CD20), Alacizumab pegol (VEGFR2), Altumomab pentetate (CEA), Amatuximab (MORAb- 009), Anatumomab mafenatox (TAG-72), Apolizumab (HLA-DR), Arcitumomab (CEA), Atezolizumab (PD-L1), Bavituximab (phosphatidylserine), Bectumomab (CD22), Belimumab (BAFF), Bevacizumab (VEGF-A), Bivatuzumab mertansine (CD44 v6), Blinatumomab (CD 19), Brentuximab vedotin (CD30 TNFRSF8), Cantuzumab mertansin (mucin CanAg), Cantuzumab ravtansine (MUC1), Capromab pendetide (prostatic carcinoma cells), Carlumab (CNT0888), Catumaxomab (EpCAM, CD3), Cetuximab (EGFR), Citatuzumab bogatox (EpCAM), Cixutumumab (IGF-1 receptor), Claudiximab (Claudin), Clivatuzumab tetraxetan (MUC1), Conatumumab (TRAIL-R2), Dacetuzumab (CD40), Dalotuzumab (insulin-like growth factor I receptor), Denosumab (RANKL), Detumomab (B-lymphoma cell), Drozitumab (DR5), Ecromeximab (GD3 ganglioside), Edrecolomab (EpCAM), Elotuzumab (SLAMF7), Enavatuzumab (PDL192), Ensituximab (NPC-1C), Epratuzumab (CD22), Ertumaxomab (HER2/neu, CD3), Etaracizumab (integrin αvβ3), Farletuzumab (folate receptor 1), FBTA05 (CD20), Ficlatuzumab (SCH 900105), Figitumumab (IGF-1 receptor), Flanvotumab (glycoprotein 75), Fresolimumab (TGF-β), Galiximab (CD80), Ganitumab (IGF-I), Gemtuzumab ozogamicin (CD33), Gevokizumab (ILIβ), Girentuximab (carbonic anhydrase 9 (CA-IX)), Glembatumumab vedotin (GPNMB), Ibritumomab tiuxetan (CD20), Icrucumab (VEGFR-1 ), Igovoma (CA-125), Indatuximab ravtansine (SDC1), Intetumumab (CD51), Inotuzumab ozogamicin (CD22), Ipilimumab (CD 152), Iratumumab (CD30), Labetuzumab (CEA), Lexatumumab (TRAIL-R2), Libivirumab (hepatitis B surface antigen), Lintuzumab (CD33), Lorvotuzumab mertansine (CD56), Lucatumumab (CD40), Lumiliximab (CD23), Mapatumumab (TRAIL-R1), Matuzumab (EGFR), Mepolizumab (IL5), Milatuzumab (CD74), Mitumomab (GD3 ganglioside), Mogamulizumab (CCR4), Moxetumomab pasudotox (CD22), Nacolomab tafenatox (C242 antigen), Naptumomab estafenatox (5T4), Namatumab (RON), Necitumumab (EGFR), Nimotuzumab (EGFR), Nivolumab (IgG4), Ofatumumab (CD20), Olaratumab (PDGF-R a), Onartuzumab (human scatter factor receptor kinase), Oportuzumab monatox (EpCAM), Oregovomab (CA-125), Oxelumab (OX-40), Panitumumab (EGFR), Patritumab (HER3), Pemtumoma (MUC1), Pertuzuma (HER2lneu), Pintumomab (adenocarcinoma antigen), Pritumumab (vimentin), Racotumomab (N- glycolylneuraminic acid), Radretumab (fibronectin extra domain-B), Rafivirumab (rabies virus glycoprotein), Ramucirumab (VEGFR2), Rilotumumab (HGF), Rituximab (CD20), Robatumumab (IGF-1 receptor), Samalizumab (CD200), Sibrotuzumab (FAP), Siltuximab (IL6), Tabalumab (BAFF), Tacatuzumab tetraxetan (alpha-fetoprotein), Taplitumomab paptox (CD 19), Tenatumomab (tenascin C), Teprotumumab (CD221), Ticilimumab (CTLA- 4), Tigatuzumab (TRAIL-R2), TNX-650 (IL13), Tositumomab (CD20), Trastuzumab (HER2/neu), TRBS07 (GD2), Tremelimumab (CTLA-4), Tucotuzumab celmoleukin (EpCAM), Ublituximab (MS4A1), Urelumab (4-1 BB), Volociximab (integrin α5β1), Votumumab (tumor antigen CTAA 16.88), Zalutumumab (EGFR), and Zanolimumab (CD4).

Citation of documents and studies referenced herein is not intended as an admission that any of the foregoing is pertinent prior art. All statements as to the contents of these documents are based on the information available to the applicants and do not constitute any admission as to the correctness of the contents of these documents.

The following description is presented to enable a person of ordinary skill in the art to make and use the various embodiments. Descriptions of specific devices, techniques, and applications are provided only as examples. Various modifications to the examples described herein will be readily apparent to those of ordinary skill in the art, and the general principles defined herein may be applied to other examples and applications without departing from the spirit and scope of the various embodiments. Thus, the various embodiments are not intended to be limited to the examples described herein and shown, but are to be accorded the scope consistent with the claims.

### Examples

### Examples:

### Methods:

### Animals

C57BL/6BrdCrHsd-*Tyr^{c}* mice were purchased from Envigo Labs. Age (8-10 weeks old) and sex (male or female) matched animals were used throughout the experiments. Congenic C57BI/6-Thy1.1 mice were bred in the animal facility of the BioNTech AG, Germany.

### CAR construct / CAR T cells

A gamma-retroviral self-inactivating (SIN) vector pES.12-6 was used to stably overexpress the CLDN6-CAR-BBz-T2A-Luc-T2A-GFP in murine T cells under the control of an internal eukaryotic promoter, the short intron-less version of the human elongation factor 1-alpha promoter (EFS -213/+31). The vector backbone contains the MLV wild type sequences of the R- und U5-regions at the 5' and 3'-LTRs as well as the packaging region (psi and psi+). The enhancer elements in the U3-region of the 3'-LTR were eliminated (including CAAT-Box), and the TATA-Box sequence was mutated to prevent transcription initiation. The truncated version of the post-transcriptional regulatory element (PRE) of the Woodchuck Hepatitis Virus (WHV) is used to prevent the expression of unwanted viral proteins. The CLDN6-CAR-BBz comprises a signaling peptide of human IgG (SEQ ID NO: 12), a single chain Fv-fragment of the Claudin6-specific antibody IMAB206 (Ganymed Pharmaceuticals) with a (G₄S)₃ linker (SEQ ID NO: 14) between the heavy (V_{H}) (SEQ ID NO: 13) and the light (V_{L}) (SEQ ID NO: 15) chain with cysteine to serine substitution at position 46 of the V_{L}. ScFv fragment is fused to human CD8α hinge and transmembrane region (SEQ ID NO: 16) followed by human 4-1BB (SEQ ID NO: 17) and human CD3ζ (Q14K) (SEQ ID NO: 18) signaling moieties. The CAR is linked with T2A ribosomal skip elements (SEQ ID NO: 19) to effective firefly luciferase (SEQ ID NO: 20) as well as to eGFP (SEQ ID NO: 21) enabling equimolar production of indicated proteins in transduced T cells.

### Retroviral gene manipulation and preparation of CAR T cells for adoptive T cell transfer

Splenocytes of naive C57BI/6-Thy1.1+ were isolated and pre-activated by Dynabeads^{™} Mouse T-Activator CD3/CD28 in a bead to T cell ratio of 1:1 (Invitrogen) in the presences of 5 ng/mL recombinant human (rh) IL-7 and 5 ng/mL rh IL-15 (Miltenyi Biotec). For transduction of murine cells, MLV-E-pseudotyped retroviral supernatants were loaded onto RetroNectin (2 µg/cm²)-coated non-tissue culture treated well plates according to the manufacturer's instruction (Takara Bio Inc., Otsu, Japan), with 3 repeated cycles of virus loading and centrifugation (1,300 xg, 15 °C, 15 min) for increased binding. 24h after pre-activation, 0.5-0.6×10^6 cells/cm² has been spun down (300 xg, 37°C, 1h) onto viral particle coated wells. After overnight cultivation, spin-down transduction was repeated with freshly viral particles coated plates. 72h after pre-activation, Dynabeads^{™} Mouse T-Activator CD3/CD28 were removed from culture and cells were expanded in the presence of 5 ng/mL rh IL-7 and 5 ng/mL rh IL-15. After ficoll cleaning, cells were washed twice with PBS to remove serum proteins and were then prepared for adoptive cell transfer (ACT). pES12.6 based retroviral vectors containing either OT1-TCR or CLDN6-CAR encoded as well enhanced firefly luciferase (effLuc; Rabinovich et al. (2008) PNAS 105(38):14342-6) and eGFP (enhanced green fluorescence protein) reporter gene, which expressed separately using 2A-splice elements (Szymczak et al. (2004) Nat Biotechnol. 22(5):589-94) were used for transduction.

### Generation of in vitro transcribed (IVT) mRNA

*In vitro* transcriptions of cytokine-albumin fusion protein encoding mRNAs were based on the pST4-T7-GG-TEV-MCS-FI-A30LA70 plasmid backbones and derivative DNA-constructs. These plasmid constructs contain the 5' leader sequence of tobacco etch virus (TEV), a 3' Fl element (where F is a 136 nucleotide long 3' -UTR fragment of amino-terminal enhancer of split, mRNA and I is a 142 nucleotide long fragment of mitochondrially encoded 12S RNA both identified in Homo sapiens; WO 2017/060314) and a poly(A) tail of 100 nucleotides, with a linker after 70 nucleotides.. Cytokine and serum albumin coding sequences originate from *Mus musculus* and no changes in the resulting amino acid sequences were introduced (mouse (m)IL-2, SEQ ID NO: 5 , mIL-7, SEQ ID NO: 6 and mIL-21, SEQ ID NO: 7). Encoded proteins are equipped with an N-terminal signal peptide (SP) that is the native SP of N-terminal moiety. Only the SP of the N-terminal moiety was maintained, for further moieties only the mature portion (protein without SP) was encoded. The stop codon was maintained for the most C-terminal moiety only. The albumin and cytokine moieties in the constructs were separated by a 30-nucleotide long linker sequence encoding for glycine and serine residues. The orientation of the used albumin-cytokine fusion proteins were the follows: albumin-linker-mIL2 (consecutive from N to C-terminus SEQ ID NOs: 8, 9 and 10), mIL7-linker-albumin (consecutive from N to C-terminus SEQ ID NOs: 6, 9 and 11) and mlL21-linker-albumin (consecutive from N to C-terminus SEQ ID NOs: 7, 9, and 11). *In vitro* transcriptions of antigen encoding mRNAs were based on the pST1-T7-GG-hAg-MCS-2hBg-A30LA70 plasmid backbones and derivative DNA-constructs. These plasmid constructs contains beside the full length human CLDN6 or chicken Ovalbumin-epitope SIINFEKL (Oval; additionally flanked with a 3' Sec and a 5' TM1 -sequence as described in Kreiter et al (2008) J Immunol. 180(1):309-18) the 5' human α-globin, two serial 3' human β-globin UTR and a poly(A) tail of 100 nucleotides, with a linker after 70 nucleotides, Antigen and cytokine encoding mRNA were generated by *in vitro* transcription as described by Holtkamp S. et al. (2006) Blood 108(13):4009-17. The latter was additionally modified by substitution of the normal nucleoside uridine by 1-methyl-pseudouridine. Resulting cytokine mRNAs were equipped with a Cap1-structure and double stranded (dsRNA) molecules were depleted by cellulose purification. Purified mRNA was eluted in H₂O and stored at - 80°C until further use. *In vitro* transcription of all described mRNA constructs was carried out at BioNTech RNA Pharmaceuticals GmbH.

### Generation of liposomal formulated antigen coding IVT RNA (RNA_{(LIP)})

Complexing of antigen encoding IVT RNA with liposomes was previously described in Kranz et al (2016) Nature 534(7607):396-401. A charge ratio of 1.3 to 2 of cationic DOTMA and RNA was used. Besides DOTMA the lipid fraction does contain the helper lipid DOPE in a molar ratio of 2:1 DOTMA per DOPE.

### Mouse experiments

5×10^6 gamma-retroviral transduced CAR or TCR congenic Thy1.1+ T cells were intravenously (i.v.) transferred in 200 µL into either immunocompetent or moderately total body irradiated (2.5 Gy - XRAD320) C57BL/6BrdCrHsd-*Tyr^{c}* donor mice. Subsequently, mice were intravenously (*i.v*.) vaccinated with an F12:RNA ratio of 1.3 : 2 of antigen encoding RNA_{(LIP)} at various time points after ACT. At indicated time points mice were treated repetitively with 1 µg nucleoside modified murine albumin-cytokine fusion protein encoded mRNA formulated with TransiT (Mirrus) or buffer alone. Peripheral blood donation and whole body bioluminescence imaging were performed at indicated time points.

### In vivo luciferase imaging (BLI)

Expansion and distribution of CAR or TCR-effLuc-GFP transduced T cells were evaluated by *in vivo* bioluminescence imaging using the IVIS Lumina imaging system (Caliper Life Sciences). Briefly, an aqueous solution of D-luciferin (80 mg/kg body weight; Perkin Elmer) was injected i.p. at indicated time points after adoptive transfer of transduced T cells. 5 min thereafter, emitted photons were quantified (integration time of 1 min, binning 8). *In vivo* bioluminescence in regions of interest (ROI) were quantified as total flux (photons/sec) using IVIS Living Image 4.0 Software. The intensity of transmitted light originating from luciferase expressing cells within the animal was represented as a greyscale image, where black is the least intense and white to dark-grey the most intense bioluminescence signal. Greyscale reference images of mice were obtained under LED low light illumination. The images were superimposed using the Living Image 4.0 software.

### Example 1: Selected pharmacokinetic-extended gamma chain cytokines (IL-2/7) lead to repetitive expansion of CAR T cells in vivo upon antigen contact.

Usually, a certain cytokine milieu needs to be present to maintain T cell persistence upon antigen contact. It has been shown that gamma chain cytokines, for instance IL-2 and IL-7 enhance the proliferation and survival of T cells (e.g. Blattman et al. (2003) Nat. Med. 9(5):540-7, Fry et al. (2001) Trends Immunol. 22(10):564-71, Bradley et al. (2005) Trends Immunol. 26(3):172-6, Jiang et al. (2005) Cytokine Growth Factor Rev. 16(4-5):513-33). The use of recombinant cytokines, such as IL-2, has been however limited by its short half-life as well as its dose-dependent toxicity (Vial et al. (1992) Drug Saf. 7(6):417-33). To overcome the limited cytokine support of adoptively transferred T cells cytokine-albumin fusion protein encoding mRNA constructs were developed and could indeed significantly increase serum half-lives of the encoded cytokines *in vivo* upon systemic administration. The systemic availability of cytokine-albumin constructs is prolonged when they are encoded on nucleoside-modified mRNA.

We therefore asked the question whether a combination of liposomally formulated TAA e.g. CLDN6-encoding RNA (RNA_{(LIP)}), which selectively targets APCs in secondary lymphoid organs and selected cytokines support can lead to an adequate repetitive expansion and persistence of CAR-T cells *in vivo.* To test this concept, gamma-retrovirally transduced CLDN6-CAR T cells were adoptively transferred to either moderately irradiated (2.5 Gy) or immunocompetent mice **(****Figure 1** **and** **2** **respectively).** In order to visualize the expansion and fate of those murine CLDN6-CAR T cells *in vivo* we took advantage of coexpression of luciferase and GFP reporter on the same CLDN6 CAR encoding retroviral vector separated by viral T2A sequences **(****Figure 1A****).** Of note, surface expression and antigen specificity of CLDN6-CAR was not significantly affected by co-expression of luciferase and GFP in CAR-transduced murine T cells (data not shown).

Moderately irradiated (2.5 Gy with XRAD320) albino C57BI/6 mice were engrafted with 5 × 10⁶ CLDN6-CAR-reporter transduced congenic Thy1.1⁺murine bulk T cells (approx. 2.5 × 10⁸ cells/kg body weight). 20 µg CLDN6-encoding or control RNA were formulated into spleen targeting liposomes and were intravenously injected into mice 1 day after adoptive CAR-T transfer. Concomitant with the CLDN6 RNA_{(LIP)-}vaccination, mice received intraperitoneally albumin coupled murine IL-2 and murine IL-7-encoding mRNA formulated in TransiT (1 µg/cytokine RNA) or mock control (buffer). Treatment was repeated 7 days later. At indicated time points CAR-T expansion and biodistribution were then tracked *in vivo* by intraperitoneal administration of 1.66 mg D-Luciferin solution per mouse. 24 hours after ACT, most of the CAR-T cells were already found in the spleen. In the absence of cytokines (mock) a ~21 fold increase (compared to day 1) in CAR-T cells was induced exclusively by treatment with CLDN6-RNA_{(LIP)} as detected in bioluminescence at day 4 after ACT. The second boost with CLDN6-RNA_{(LIP)} resulted at day 11 still in an 15 fold higher luminescence intensity compared to baseline luminescence measured at day 1. Expansion capacity of CAR T cells increased significantly, when TransiT formulated albumin fusion IL-2 and IL-7-encoding mRNA were co-administered. A 75 fold expansion of CAR-T cells could be achieved after the first RNA_{(LIP)} treatment and has been even improved with the second CLDN6 RNA_{(LIP)} up to 114 fold **(****Figure 1C**&D). This effect was observed for mice having received CLDN6-CAR T cells after treatment with CLDN6-encoding RNA_{(LIP)} alone or in combination with cytokine-albumin encoding RNAs, but not in the respective control groups which received Oval encoding control RNA_{(LIP)} neither in the presence nor absence of cytokine-albumin encoding RNAs. These data demonstrate that CAR-T cells can successfully be expanded *in situ* in a highly antigen-specific manner in moderately irradiated mice.

After having demonstrated that CAR T cells can repetitive expanded *in situ* using RNA_{(LIP)} encoding the respective antigen in the presence of cytokine encoding RNAs in moderately irradiated mice, we investigated whether this effect can be also achieved in immunocompetent host. Since, lympho-depletion has, however, several disadvantages including the well-known side effects and risks associated with chemotherapies such as potential infections and sepsis (Brentjens et al. (2010) Mol Ther. 18(4):666-8 & Robbins et al. (2015) Clin Cancer Res. 21(5):1019-27). Furthermore - in cases of on- and/or off-target toxicities - rapid expansion of adoptively transferred CAR-T cells could be fatal (Morgan et al. (2010) Mol Ther. 18(4):843-51). For this purpose CLDN6-CAR transduced murine Thy1.1+ T cell-engrafted non-irradiated albino C57BI/6 mice were treated as described above **(****Figure 2A****).** The systemic presence of IL-2 und IL-7 during the first stimulation round has no significant impact on the expansion of CLDN6-CAR T cells after CLDN6-RNA_{(LIP)} vaccination compared to the control group, which receive buffer (mock) instead of TransiT-formulated cytokine-albumin encoding RNAs (day 4: expansion index: mock 192 fold and IL-2/7: 223 fold). However, in the absence of IL-2/7 cytokines, CAR T cell population strongly contracted after first CLDN6 RNA_{(LIP)} mediated expansion and could not re-expand a second time. Only in the presence of IL-2/IL-7-albumin encoding RNA CLDN6-CAR T cells can be repetitive expand and persists over several days in immunocompetent mice (day 11: expansion index: mock: 0.5 fold and IL-2/7: 79 fold) (**Figure 2B****+C**).

These data strongly support the idea that controlled CAR-T cell expansion directly in the patient using RNA_{(LIP)} technology is feasible, but for persistence the cells need a favorable cytokine milieu, such as IL-2 and IL-7, which can be achieved by administration of RNA encoding extended pharmacokinetic gamma chain cytokines.

### Example 2: Optimal combination of cytokine albumin fusions during repetitive expansion of CAR T cells.

Since several gamma chain cytokines positively support the T cell survival and support the therapeutic effects of T cells in an antigen specific manner (e.g. Markley et al. (2010) Blood 115(17):3508-19, He et al. (2006) J Transl Med. 4:24.), we compared nucleoside-modified RNA encoding mIL-2, mIL-7, mIL-21 and the combination IL-2/7 and IL-2/21 in terms of facilitating supportive effects of proliferation and persistence of CAR modified T cells *in vivo* upon repetitive RNA_{(LIP)} treatment.

In a similar manner as described in Example 1, moderately irradiated albino C57BI/6 mice with transplanted CLDN6-CAR-reporter transduced T cells were vaccinated with liposomally formulated hCLDN6 or ctrl encoding RNA concomitantly with the treatment of RNA encoding murine albumin coupled mlL-2, mlL-7, mlL-21-encoding mRNA formulated in TransIT (1 µg/cytokine RNA) or murine albumin encoding RNA (Alb ctrl). Antigen/cytokine cocktail was administered with an interval of one week in between (**Figure 3A**). At peak of *in vivo* expansion of CAR T cells (usually reached after 2-3 days post RNA based treatment), the bioluminescence intensity was analyzed (**Figure 3B**). The systemically presence of IL-7 and IL-21 alone resulted in reduced antigen-specific CART expansion capabilities upon repetitive RNA_{(LIP)} treatment compared to albumin control. IL-2 co-treatment resulted in up to 164 fold CAR T cell expansion compared when compared to baseline. However an *in vivo* accumulation of CAR T cells could only be achieved, when IL-2 RNA was co-administered with IL-7 (up to 214 fold increase after 3^{rd} expansion round) or IL-21 (up to 141 fold increase upon after 3^{rd} expansion round) respectively. Beside the accumulation capability of CAR T cells *in vivo,* the clinical success of adoptively transferred tumor reactive T cell therapy has been also positively correlated with the persistence of those cells *in vivo* (Robbins et al. (2004) J Immunol. 173(12):7125-30, Huang et al. (2005) 28(3):258-67). We therefore analyzed the contraction of CART T cells after 3 rounds of antigen specific expansion using bioluminescence in the presence of IL-7 **(****Figure 3C****)** or in the presence of IL-21 **(****Figure 3D****)** either alone or in combination with IL-2. Whereas the CAR T cell population contracted shortly after 3^{rd} CLDN6 RNA_{(LIP)} when exclusively albumin, IL-2 or IL-7 were present. However, only the combination of IL-2 and IL-7 can augment decelerated contraction of CLDN6 CAR T cells after antigen withdrawal **(****Figure 3C****).** The effect was even more distinctive in IL-2 and IL-21- RNA co-treated mice **(****Figure 3D****).**

Overall, these results indicate that systemic administration of nucleoside-modified RNA encoding IL-2 and in the commitment with IL-7 and IL-21 can augment highly antigen-dependent accumulation and prolonged persistence of CAR T cells *in vivo* upon antigen specific stimulation.

The invention provides, in particular, the following:
1. A method for inducing an immune response in a subject comprising:
   a. providing to the subject T cells genetically modified to express a chimeric antigen receptor (CAR) and
   b. administering to the subject IL2 or a polynucleotide encoding IL2.
2. The method of item 1, which comprises administering IL2 or a polynucleotide encoding IL2 and a further cytokine or a polynucleotide encoding a further cytokine.
3. The method of item 2, wherein the further cytokine is selected from the group consisting of IL7 and IL21.
4. The method of any one of items 1 to 3, which comprises administering IL2 or a polynucleotide encoding IL2 and IL7 or a polynucleotide encoding IL7.
5. The method of any one of items 1 to 3, which comprises administering IL2 or a polynucleotide encoding IL2 and IL21 or a polynucleotide encoding IL21.
6. The method of any one of items 1 to 5, wherein the polynucleotide encoding IL2 is RNA and optionally the polynucleotide encoding a further cytokine is RNA.
7. The method of any one of items 1 to 6, wherein the T cells genetically modified to express a CAR are provided to the subject by administering the T cells genetically modified to express a CAR or by generating the T cells genetically modified to express a CAR in the subject.
8. The method of any one of items 1 to 7, which further comprises administering to the subject an antigen or a variant thereof, or a polynucleotide encoding the antigen or variant, wherein the T cells genetically modified to express a CAR are targeted to the antigen and the immune response is an immune response to a target cell population or target tissue expressing the antigen.
9. The method of item 8, wherein the polynucleotide encoding the antigen or variant is RNA.
10. A method for inducing an immune response in a subject comprising:
   a. providing to the subject T cells genetically modified to express a chimeric antigen receptor (CAR) and
   b. administering to the subject RNA encoding IL2.
11. The method of item 10, which comprises administering RNA encoding IL2 and RNA encoding a further cytokine.
12. The method of item 11, wherein the further cytokine is selected from the group consisting of IL7 and IL21.
13. The method of any one of items 10 to 12, which comprises administering RNA encoding IL2 and RNA encoding IL7.
14. The method of any one of items 10 to 12, which comprises administering RNA encoding IL2 and RNA encoding IL21.
15. The method of any one of items 10 to 14, wherein the T cells genetically modified to express a CAR are provided to the subject by administering the T cells genetically modified to express a CAR or by generating the T cells genetically modified to express a CAR in the subject.
16. The method of any one of items 10 to 15, which further comprises administering to the subject RNA encoding an antigen or a variant thereof, wherein the T cells genetically modified to express a CAR are targeted to the antigen and the immune response is an immune response to a target cell population or target tissue expressing the antigen.
17. The method of any one of items 1 to 16, wherein the immune response is a T cell-mediated immune response.
18. A method for treating a subject having a disease, disorder or condition associated with expression or elevated expression of an antigen comprising:
   a. providing to the subject T cells genetically modified to express a chimeric antigen receptor (CAR) targeted to the antigen and
   b. administering to the subject IL2 or a polynucleotide encoding IL2.
19. The method of item 18, which comprises administering IL2 or a polynucleotide encoding IL2 and a further cytokine or a polynucleotide encoding a further cytokine.
20. The method of item 19, wherein the further cytokine is selected from the group consisting of IL7 and IL21.
21. The method of any one of items 18 to 20, which comprises administering IL2 or a polynucleotide encoding IL2 and IL7 or a polynucleotide encoding IL7.
22. The method of any one of items 18 to 20, which comprises administering IL2 or a polynucleotide encoding IL2 and IL21 or a polynucleotide encoding IL21.
23. The method of any one of items 18 to 22, wherein the polynucleotide encoding IL2 is RNA and optionally the polynucleotide encoding a further cytokine is RNA.
24. The method of any one of items 18 to 23, wherein the T cells genetically modified to express a CAR are provided to the subject by administering the T cells genetically modified to express a CAR or by generating the T cells genetically modified to express a CAR in the subject.
25. The method of any one of items 18 to 24, which further comprises administering to the subject the antigen or a variant thereof, or a polynucleotide encoding the antigen or variant.
26. The method of item 25, wherein the polynucleotide encoding the antigen or variant is RNA.
27. A method for treating a subject having a disease, disorder or condition associated with expression or elevated expression of an antigen comprising:
   a. providing to the subject T cells genetically modified to express a chimeric antigen receptor (CAR) targeted to the antigen and
   b. administering to the subject RNA encoding IL2.
28. The method of item 27, which comprises administering RNA encoding IL2 and RNA encoding a further cytokine.
29. The method of item 28, wherein the further cytokine is selected from the group consisting of IL7 and IL21.
30. The method of any one of items 27 to 29, which comprises administering RNA encoding IL2 and RNA encoding IL7.
31. The method of any one of items 27 to 29, which comprises administering RNA encoding IL2 and RNA encoding IL21.
32. The method of any one of items 27 to 31, wherein the T cells genetically modified to express a CAR are provided to the subject by administering the T cells genetically modified to express a CAR or by generating the T cells genetically modified to express a CAR in the subject.
33. The method of any one of items 27 to 32, which further comprises administering to the subject RNA encoding the antigen or a variant thereof.
34. The method of any one of items 18 to 33, wherein the disease, disorder or condition is cancer and the antigen is a tumor-associated antigen.
35. The method of any one of items 1 to 34, wherein IL2 is extended pharmacokinetic (PK) IL2.
36. The method of item 35, wherein the extended-PK IL2 comprises a fusion protein.
37. The method of item 36, wherein the fusion protein comprises an IL2 moiety and a moiety selected from the group consisting of serum albumin, an immunoglobulin fragment, transferrin, Fn3, and variants thereof.
38. The method of any one of items 2 to 9, 11 to 17, 19 to 26 and 28 to 37, wherein the further cytokine, in particular IL7 or IL21, is extended pharmacokinetic (PK) cytokine, in particular extended-PK IL7 or extended-PK IL21.
39. The method of item 38, wherein the extended-PK cytokine, in particular extended-PK IL7 or extended-PK IL21, comprises a fusion protein.
40. The method of item 39, wherein the fusion protein comprises a moiety of the further cytokine, in particular an IL7 moiety or an IL21 moiety, and a moiety selected from the group consisting of serum albumin, an immunoglobulin fragment, transferrin, Fn3, and variants thereof.
41. The method of any one of items 37 to 40, wherein the serum albumin comprises mouse serum albumin or human serum albumin.
42. The method of any one of items 37 to 41, wherein the immunoglobulin fragment comprises an immunoglobulin Fc domain.
43. The method of any one of items 1 to 42, which is a method for treating or preventing cancer in a subject, wherein the antigen is a tumor-associated antigen.
44. A medical preparation comprising:
   a. T cells genetically modified to express a chimeric antigen receptor (CAR) and
   b. IL2 or a polynucleotide encoding IL2.
45. The medical preparation of item 44, which comprises IL2 or a polynucleotide encoding IL2 and a further cytokine or a polynucleotide encoding a further cytokine.
46. The medical preparation of item 45, wherein the further cytokine is selected from the group consisting of IL7 and IL21.
47. The medical preparation of any one of items 44 to 46, which comprises IL2 or a polynucleotide encoding IL2 and IL7 or a polynucleotide encoding IL7.
48. The medical preparation of any one of items 44 to 46, which comprises IL2 or a polynucleotide encoding IL2 and IL21 or a polynucleotide encoding IL21.
49. The medical preparation of any one of items 44 to 48, wherein the polynucleotide encoding IL2 is RNA and optionally the polynucleotide encoding a further cytokine is RNA.
50. The medical preparation of any one of items 44 to 49, which further comprises an antigen or a variant thereof, or a polynucleotide encoding the antigen or variant, wherein the T cells genetically modified to express a CAR are targeted to the antigen.
51. The medical preparation of item 50, wherein the polynucleotide encoding the antigen or variant is RNA.
52. The medical preparation of any one of items 44 to 51, which is a kit.
53. The medical preparation of item 52, which comprises the T cells genetically modified to express a CAR, the IL2 or the polynucleotide encoding IL2, optionally the further cytokine or the polynucleotide encoding a further cytokine and optionally the antigen or a variant thereof, or the polynucleotide encoding the antigen or variant in separate containers.
54. The medical preparation of item 52 or 53, further comprising instructions for use of the medical preparation for treating or preventing cancer wherein the antigen is a tumor-associated antigen.
55. The medical preparation of any one of items 44 to 51, which is a pharmaceutical composition.
56. The medical preparation of item 55, wherein the pharmaceutical composition further comprises one or more pharmaceutically acceptable carriers, diluents and/or excipients.
57. A medical preparation comprising:
   a. T cells genetically modified to express a chimeric antigen receptor (CAR) and
   b. RNA encoding IL2.
58. The medical preparation of item 57, which comprises RNA encoding IL2 and RNA encoding a further cytokine.
59. The medical preparation of item 58, wherein the further cytokine is selected from the group consisting of IL7 and IL21.
60. The medical preparation of any one of items 57 to 59, which comprises RNA encoding IL2 and RNA encoding IL7.
61. The medical preparation of any one of items 57 to 60, which comprises RNA encoding IL2 and RNA encoding IL21.
62. The medical preparation of any one of items 57 to 61, which further comprises RNA encoding an antigen or a variant thereof, wherein the T cells genetically modified to express a CAR are targeted to the antigen.
63. The medical preparation of any one of items 57 to 62, which is a kit.
64. The medical preparation of item 63, which comprises the T cells genetically modified to express a CAR, the RNA encoding IL2, optionally the RNA encoding a further cytokine, and optionally the RNA encoding an antigen or a variant thereof in separate containers.
65. The medical preparation of item 63 or 64, further comprising instructions for use of the medical preparation for treating or preventing cancer wherein the antigen is a tumor-associated antigen.
66. The medical preparation of any one of items 57 to 62, which is a pharmaceutical composition.
67. The medical preparation of item 66, wherein the pharmaceutical composition further comprises one or more pharmaceutically acceptable carriers, diluents and/or excipients.
68. The medical preparation of any one of items 44 to 67, wherein IL2 is extended pharmacokinetic (PK) IL2.
69. The medical preparation of item 68, wherein the extended-PK IL2 comprises a fusion protein.
70. The medical preparation of item 69, wherein the fusion protein comprises an IL2 moiety and a moiety selected from the group consisting of serum albumin, an immunoglobulin fragment, transferrin, Fn3, and variants thereof.
71. The medical preparation of any one of items 45 to 56 and 58 to 70, wherein the further cytokine is extended pharmacokinetic (PK) cytokine.
72. The medical preparation of item 71, wherein the extended-PK cytokine comprises a fusion protein.
73. The medical preparation of item 72, wherein the fusion protein comprises a cytokine moiety and a moiety selected from the group consisting of serum albumin, an immunoglobulin fragment, transferrin, Fn3, and variants thereof.
74. The medical preparation of any one of items 70 to 73, wherein the serum albumin comprises mouse serum albumin or human serum albumin.
75. The medical preparation of any one of items 70 to 74, wherein the immunoglobulin fragment comprises an immunoglobulin Fc domain.
76. The medical preparation of any one of items 44 to 75 for pharmaceutical use.
77. The medical preparation of item 76, wherein the pharmaceutical use comprises a therapeutic or prophylactic treatment of a disease or disorder.
78. The medical preparation of any one of items 44 to 77 for use in a method for treating or preventing cancer in a subject, wherein the antigen is a tumor-associated antigen.

## Claims

1. A medical preparation comprising:
a. IL2 or a polynucleotide encoding IL2, and
b. a further cytokine or a polynucleotide encoding the further cytokine, wherein the further cytokine is selected from the group consisting of IL7 and IL21,
for use in a method for inducing an immune response in a subject, the method comprising providing to the subject T cells genetically modified to express a chimeric antigen receptor (CAR).

2. The medical preparation for use of claim 1, wherein the polynucleotide encoding IL2 is RNA and optionally the polynucleotide encoding the further cytokine is RNA.

3. The medical preparation for use of claim 1 or 2, which further comprises an antigen or a variant thereof, or a polynucleotide encoding the antigen or variant, wherein the T cells genetically modified to express a CAR are targeted to the antigen.

4. The medical preparation for use of claim 3, wherein the polynucleotide encoding the antigen or variant is RNA.

5. The medical preparation for use of any one of claims 1 to 4, which is a kit.

6. The medical preparation for use of claim 5, which comprises the IL2 or the polynucleotide encoding IL2, the further cytokine or the polynucleotide encoding a further cytokine and optionally the antigen or a variant thereof, or the polynucleotide encoding the antigen or variant in separate containers, and which optionally comprises instructions for use of the medical preparation for treating or preventing cancer wherein the antigen is a tumor-associated antigen.

7. The medical preparation for use of any one of claims 1 to 6, which is a pharmaceutical composition, wherein, preferably, the pharmaceutical composition further comprises one or more pharmaceutically acceptable carriers, diluents and/or excipients.

8. The medical preparation for use of any one of claims 1 to 7, wherein IL2 is extended pharmacokinetic (PK) IL2, wherein, preferably, the extended-PK IL2 comprises a fusion protein, the fusion protein optionally comprising an IL2 moiety and a moiety selected from the group consisting of serum albumin, an immunoglobulin fragment, transferrin, Fn3, and variants thereof.

9. The medical preparation for use of any one of claims 1 to 8, wherein the further cytokine is extended pharmacokinetic (PK) cytokine, wherein, preferably, the extended-PK cytokine comprises a fusion protein.

10. The medical preparation for use of claim 8 or 9, wherein the fusion protein comprises a cytokine moiety and a moiety selected from the group consisting of serum albumin, an immunoglobulin fragment, transferrin, Fn3, and variants thereof.

11. The medical preparation for use of claim 10, wherein the serum albumin comprises mouse serum albumin or human serum albumin.

12. The medical preparation for use of claim 10, wherein the immunoglobulin fragment comprises an immunoglobulin Fc domain.

13. The medical preparation for use of any one of claims 1 to 12, wherein providing to the subject T cells genetically modified to express a CAR comprises transfecting T cells *in vivo* with a nucleic acid encoding the CAR.

14. The medical preparation for use of any one of claims 1 to 13, wherein the method comprises a therapeutic or prophylactic treatment of a disease or disorder.

15. The medical preparation for use of any one of claims 1 to 14 wherein the method is a method for treating or preventing cancer in a subject, wherein the cancer is associated with expression or elevated expression of a tumor-associated antigen.
